# EUROPEAN PATENT SPECIFICATION

(11) **EP 4 118 197 B1**
(45) Date of publication and mention of the grant of the patent: **23.10.2024**
(21) Application number: 21719308.5
(22) Date of filing: 11.03.2021
(51) Int. Cl.: C12N 9/22

(54) **HIGH FIDELITY SPCAS9 NUCLEASES FOR GENOME MODIFICATION**
HIGH FIDELITY SPCAS9 NUKLEASES ZUR GENMODIFIKATION
SPCAS9 NUCLÉASES DE HAUTE FIDÉLITÉ POUR LA MODIFICATION DU GÉNOME

(30) Priority: 11.03.2020 US 202062988279 P
(43) Date of publication of application: 18.01.2023
(73) Proprietor: Sigma-Aldrich Co. LLC, St. Louis, MO 63103 (US)
(72) Inventor: CHEN, Fuqiang, St. Louis, MO 63103 (US)
(74) Representative: Greaves Brewster LLP
(86) International application number: PCT/US2021/021922
(87) International publication number: WO 2021/183771

(56) References cited:
- WO-A1-2016/205613
- WO-A1-2018/149888
- US-A1- 2018 320 201
- ANTONIO CASINI ET AL: "A highly specific SpCas9 variant is identified by in vivo screening in yeast", NATURE BIOTECHNOLOGY, vol. 36, no. 3, 29 January 2018 (2018-01-29), New York, pages 265 - 271, XP055619847, ISSN: 1087-0156, DOI: 10.1038/nbt.4066

## Description

### CROSS-REFERENCE TO RELATED APPLICATIONS

**The** present application claims the benefit of priority of US Provisional Application No. 62/988,279, filed March 11, 2020.

### SEQUENCE LISTING

The instant application contains a Sequence Listing which has been submitted electronically in ASCII format and is hereby incorporated by reference in its entirety. Said ASCII copy, created on March 11, 2021, is named P20-035_WO-PCT_SL.txt and is 49,120 bytes in size.

### FIELD OF THE DISCLOSURE

The present disclosure relates to engineered Cas9 protein variants and systems, nucleic acids encoding said protein variants and systems, and methods of making and using said protein variants and systems for genome modification.

### BACKGROUND OF THE DISCLOSURE

Streptococcus pyogenes CRISPR Cas9 (SpCas9) has gained widespread adoption as a genome editing endonuclease in many cell types and organisms. The wild-type nuclease, however, has the propensity to cause mutations at unintended genomic sites bearing a sequence similar to the target site. Several SpCas9 variants with improved specificity have been developed to alleviate this drawback. These includes eSpCas9 1.0 (K810A, K1003A, R1060A), eSpCas9 1.1 (K848A, K1003A, R1060A), SpCas9-HF1 (N497A, R661A, Q695A, Q926A), HypaCas9 (N692A, M694A, Q695A, H698A), EvoCas9 (M495V, Y515N, K526E, R661L), Sniper Cas9 (F539S, M763I, K890N), HiFi Cas9 V3 (R691A), Opti-SpCas9 (R661A and K1003H), and OptiHF-SpCas9 (Q695A, K848A, E293M, T924V and Q926A) (Slaymaker et al., Science 351, 84-88; Kleinstiver et al., Nature 523, 490-495; Chen et al. Nature 550, 407-410; Casini et al., Nature Biotechnology 36, 265-271; Lee et al., Nature Communications 9, 3048; Vakulskas et al., Nature Medicine 24, 1216-1224; Choi et al., Nature Methods 16, 722-730). WO 2018/149888 discloses high fidelity Cas9 variants and applications thereof. However, most of these variants were identified through screening in the plasmid format and their conversion to recombinant proteins for genome modification by means of ribonucleoprotein (RNP) delivery often resulted in low activities.

As the demand for SpCas9 recombinant protein in genome modification has greatly increased, there is a need for the nuclease in the recombinant protein form that can perform with improved specificity and sustained activity across different genomic sites.

### SUMMARY OF THE DISCLOSURE

Among the various aspects of the present invention is the provision of engineered Cas9 protein variants and systems including the same.

In the particular, the invention provides an engineered *Streptococcus pyogenes* Cas9 (SpCas9) protein variant having an identity of at least 99% identity with SEQ ID NO:1 and comprising the mutations selected from the following group:
K562L-R661L-K855Q;
K562Q-R661L-K855Q;
K652L-R661L-K855Q;
K652Q-R661L-K855Q;
R661L-K855Q-K1003Q;
R661L-K855Q-R1060Q;
R661L-R691L-K855Q;
R661L-R780L-K855Q;
R661L-R780Q-K855Q;
R661L-K81 0L-K855Q;
R661L-K848L-K855Q;
R661L-K81 0Q-K855Q;
R661L-K855Q-K1003L; and
R661L-K855Q-R1060L;
with reference to the amino acid numbering in the corresponding position of an unmodified mature, wild-type *Streptococcus pyogenes* Cas9 shown in SEQ ID NO:1. The mutations are preferably K652Q-R661L-K855Q.

Another aspect of the invention is directed to an engineered Cas9 system comprising the engineered Cas9 protein variants disclosed herein and at least one engineered guide RNA(s), wherein each engineered guide RNA is designed to complex with the engineered Cas9 protein variant.

Another aspect of the invention is directed to nucleic acids encoding the engineered Cas9 protein variants and systems including the same. Vectors comprising the nucleic acids are also provided.

Another aspect of the invention is directed to methods of making and using the engineered Cas9 protein variants and systems described herein.

Other objects and features will be in apparent from the appended claims.

### BRIEF DESCRIPTION OF THE DRAWINGS

**FIG. 1A** shows on-target activities of five different substitutions on the K855 residue at the HEKSite4 target site in human U-2 OS cells. K855E and K855A resulted in on-target activity reduction (Example 1). FIG. discloses SEQ ID NO: 73.
**FIG. 1B** shows off-target activities of five different substitutions on the K855 residue at a HEKSite4 off-target site in human U-2 OS cells (Example 1). FIG. discloses SEQ ID NO: 74.
**FIG. 2** shows on-target activities of different substitutions on the R661, N692, or Q695 residue at the HEKSite4 target site in human U-2 OS cells (Example 2). FIG. discloses SEQ ID NO: 73.
**FIG. 3A** shows on-target activities of triple and quadruple mutant proteins at the FANCF02 target site in human K562 cells (Example 3). FIG. discloses SEQ ID NO: 75.
**FIG. 3B** shows off-target activities of triple and quadruple mutant proteins at a FANCF02 single mismatch off-target site in human K562 cells (Example 3). FIG. discloses SEQ ID NO: 76.
**FIG. 3C** shows on-target activities of triple and quadruple mutant proteins at the HBB03 target site in human K562 cells (Example 3). FIG. discloses SEQ ID NO: 77.
**FIG. 3D** shows off-target activities of triple and quadruple mutant proteins at a HBB03 single mismatch off-target site in human K562 cells (Example 3). FIG. discloses SEQ ID NO: 78.
**FIG. 4** shows on-target activities of a selected group of mutant proteins at five different genomic sites in K562 cells (Example 4). FIG. discloses SEQ ID NOS 79-83, respectively, in order of appearance.

### DETAILED DESCRIPTION

As the demand for SpCas9 recombinant protein in genome modification has greatly increased, there is a need for the nuclease in the recombinant protein form that can perform with improved specificity and sustained activity across different genomic sites. By using a recombinant protein-based screening approach, at least two different groups of SpCas9 variants with different levels of specificity and activity have been identified. One group has an extremely high level of specificity relatively to other SpCas9 variants, but a highly varying activity between different genomic sites. The other group has balanced specificity and activity, outperforming the well-established eSpCas9 1.1 in activity and the recently developed HiFi Cas9 V3 in specificity. This group of nucleases holds great potential for broad application in genome modification in eukaryotic cells.

Previous attempts to develop high fidelity SpCas9 variants have largely relied on a certain plasmid expression-based selection scheme. These variants often show low activities when used as recombinant proteins. Without being bound to a particular theory, it is speculated that plasmid overexpression in mammalian cells could disguise the attenuated activities of these variants caused by mutations to increase the specificity. To avoid this confounding effect of plasmid overexpression, a recombinant protein-based screening approach to improve the nuclease has been employed. Moreover, in contrast to previous attempts which invariably used alanine substitutions to mutate key residues, optimal amino acid substitutions were used to maintain on-target activity while improving the specificity. These differences methodologically distinguish the presently disclosed proteins from those derived from previous SpCas9 protein engineering efforts.

The mutations and combinations thereof contain key residues presumably involved in different mechanisms underlying the Cas9 DNA substrate binding stability, while previous attempts limited the mutation combinations to key residues presumably involved in one mechanism. For instance, eSpCas9 was developed by mutating conserved positively charged amino acid residues that interact with the negatively charged phosphate backbone of the non-target strand, based on the hypothesis that those positively charged residues stabilize the non-target strand upon strand separation, and subsequently stabilizing the formation of guided RNA-target DNA heteroduplex (Slaymaker et al., Science 351, 84-88). In contrast, SpCas9-HF1 was developed by reducing hydrogen bonding or charge interaction with the phosphate backbone of the target strand (Kleinstiver et al., Nature 523, 490-495). On the other hand, HypaCas9 was derived by mutating a cluster of conserved residues (N692, M694, Q695 and H698) to alanine in the REC3 domain that presumably senses the RNA-DNA interaction and transfers this signal to trigger the conformational switch of the HNH nuclease domain (Chen et al., Nature 550, 407-410).

By employing the recombinant protein-based unique screening approach and broadening the rational design to different mechanistic combinations as disclosed herein, the present disclosure has identified at least three distinct groups of SpCas9 variants with different levels of specificity and activity.

### (I) Engineered Cas9 Proteins

One aspect of the present disclosure is directed to engineered Cas proteins. The engineered Cas protein is an engineered *Streptococcus pyogenes* Cas9 (SpCas9) protein variant having an identity of at least 99% identity with SEQ ID NO:1 and comprising the mutations selected from the following group:
K562L-R661L-K855Q;
K562Q-R661L-K855Q;
K652L-R661L-K855Q;
K652Q-R661L-K855Q;
R661L-K855Q-K1003Q;
R661L-K855Q-R1060Q;
R661L-R691L-K855Q;
R661L-R780L-K855Q;
R661L-R780Q-K855Q;
R661L-K81 0L-K855Q;
R661L-K848L-K855Q;
R661L-K81 0Q-K855Q;
R661L-K855Q-K1003L; and
R661L-K855Q-R1060L;
with reference to the amino acid numbering in the corresponding position of an unmodified mature, wild-type *Streptococcus pyogenes* Cas9 shown in SEQ ID NO:1. The mutations are preferably K652Q-R661L-K855Q.

The engineered Cas9 protein variant is from *Streptococcus pyogenes* (SpCas9). Thus, in some embodiments, the engineered Cas9 proteins described herein are SpCas9 homologues.

Wild-type Cas9 proteins comprise two nuclease domains, *i.*e., RuvC and HNH domains, each of which cleaves one strand of a double-stranded sequence. Cas9 proteins also comprise REC domains that interact with the guide RNA (*e.g*., REC1, REC2) or the RNA/DNA heteroduplex (*e.g*., REC3), and a domain that interacts with the protospacer-adjacent motif (PAM) (*i.e*., PAM-interacting domain).

As noted herein, the Cas9 proteins of the present disclosure are engineered to comprise the mutations selected from the group defined in claim 1 such that the Cas9 protein has altered activity, specificity, and/or stability. These engineered Cas9 proteins do not occur naturally.

In general, known and/or commercially available Cas9 mutants have focused on a point mutation(s) in specific regions of the protein, without regard to other regions and combinations of mutations in different regions of the protein. It has been advantageously discovered that combinations of mutations in different regions of the Cas9 protein can result in improved specificity, activity (*e.g*., on- or off- target activity), and/or other beneficial properties relative to known Cas9 mutants.

For example, the Cas9 proteins disclosed herein have at least one mutation(s) in the structural region of the protein that includes non-target DNA strand contacting residues, and/or at least one mutation(s) in the structural region of the protein that includes target DNA/guide RNA heteroduplex contacting residues, and/or at least one mutation(s) in the structural region of the protein that includes an alpha-helical lobe. For purposes of this disclosure, non-target DNA strand contacting residues include, for example, amino acids R780, K810, K848, K855, K1003, and R1060; target DNA/guide RNA heteroduplex contacting residues include, for example, amino acids R661 and R691; and alpha-helical lobe residues include, for example, amino acids K526, K562, and K652 (with reference to the numbering system of *Streptococcus pyogenes* Cas9, SpCas9). Thus, in various embodiments, the Cas9 proteins disclosed herein have at least one mutation(s) in the structural region of the protein that includes non-target DNA strand contacting residues, and at least one mutation(s) in the structural region of the protein that includes target DNA/guide RNA heteroduplex contacting residues. In other embodiments, the Cas9 proteins disclosed herein have at least one mutation(s) in the structural region of the protein that includes non-target DNA strand contacting residues, and at least one mutation(s) in the structural region of the protein that includes an alpha-helical lobe. In still other embodiments, the Cas9 proteins disclosed herein have at least one mutation(s) in the structural region of the protein that includes target DNA/guide RNA heteroduplex contacting residues, and at least one mutation(s) in the structural region of the protein that includes an alpha-helical lobe. In still other embodiments, the Cas9 proteins disclosed herein have at least one mutation(s) in the structural region of the protein that includes non-target DNA strand contacting residues, and at least one mutation(s) in the structural region of the protein that includes target DNA/guide RNA heteroduplex contacting residues, and at least one mutation(s) in the structural region of the protein that includes an alpha-helical lobe.

The Cas9 protein variants disclosed herein have a modified amino acid sequence that is identified by reference to the amino acid numbering in the corresponding position of an unmodified mature (wild-type) *Streptococcus pyogenes* Cas9 (SEQ ID NO: 1). The Cas9 protein variants disclosed herein have an identity of at least 99% identity with SEQ ID NO:1:

| ***Streptococcus pyogenes* Cas9 (wild-type)** |
|---|
|  (SEQ ID NO:1) |

For ease of reference, notations of the 20 essential amino acids and their single letter codes appear below in Table A.

**Table A: Amino Acids**

| **Amino Acid** | **Single Letter Code** |
|---|---|
| Alanine | A |
| Arginine | R |
| Asparagine | N |
| Aspartic Acid | D |
| Cysteine | C |
| Glutamine | Q |
| Glutamic Acid | E |
| Glycine | G |
| Histidine | H |
| Isoleucine | I |
| Leucine | L |
| Lysine | K |
| Methionine | M |
| Phenylalanine | F |
| Proline | P |
| Serine | S |
| Threonine | T |
| Tryptophan | W |
| Tyrosine | Y |
| Valine | V |

It will be understood that the amino acid modifications described herein utilize a nomenclature that starts with a letter referring to the amino acid (single letter code) affected and ending with a letter designating the change (single letter code), with the amino acid residue position between the two letters. For example, a hypothetical protein may have an Alanine residue at a hypothetical amino acid position 100 and would be designated A100. By way of further example, a modification of the hypothetical amino acid position 100 from an Alanine to a Valine would be designed A100V. A modification selected from two or more options can be designated with a "*I*", e.g., a modification of a hypothetical amino acid position 100 from an Alanine to a Valine OR a Serine would be designed A100V/S.

In one particular embodiment, the engineered SpCas9 protein is selected from one of the following group of variants: K562L-R661L-K855Q; K562Q-R661L-K855Q; K652L-R661L-K855Q; K652Q-R661L-K855Q; R661L-K855Q-K1003Q; and R661L-K855Q-R1060Q (with reference to the numbering system of *Streptococcus pyogenes* Cas9, SpCas9). In another particular embodiment, the engineered SpCas9 protein is selected from one of the following group of variants: K562L-R661L-K855Q; K562Q-R661L-K855Q; K652L-R661L-K855Q; and K652Q-R661L-K855Q. Thus, for example, the engineered SpCas9 protein variant could be K562L-R661L-K855Q. Alternatively, for example, the engineered SpCas9 protein variant could be K562Q-R661L-K855Q. Alternatively, for example, the engineered SpCas9 protein variant could be K652L-R661L-K855Q. Alternatively, for example, the engineered SpCas9 protein variant could be K652Q-R661L-K855Q. Alternatively, for example, the engineered SpCas9 protein variant could be R661L-K855Q-K1003Q. Alternatively, for example, the engineered SpCas9 protein variant could be R661L-K855Q-R1060Q. Members of this group of variants possess relatively balanced specificity and activity, outperforming the well-established eSpCas9 1.1 in activity and the recently developed HiFi Cas9 V3 in specificity.

In another particular embodiment, the engineered SpCas9 protein is selected from one of the following group of variants: K526L-R661L-K855Q; R661L-R691L-K855Q; R661L-R780L-K855Q; R661L-R780Q-K855Q; R661L-K810L-K855Q, and R661L-K848L-K855Q (with reference to the numbering system of *Streptococcus pyogenes* Cas9, SpCas9). Thus, for example, the engineered SpCas9 protein variant could be K526L-R661L-K855Q. Alternatively, for example, the engineered SpCas9 protein variant could be R661L-R691L-K855Q. Alternatively, for example, the engineered SpCas9 protein variant could be R661L-R780L-K855Q. Alternatively, for example, the engineered SpCas9 protein variant could be R661L-R780Q-K855Q. Alternatively, for example, the engineered SpCas9 protein variant could be R661L-K810L-K855Q. Alternatively, for example, the engineered SpCas9 protein variant could be R661L-K848L-K855Q. Members of this group of variants possess extremely high levels of specificity but highly varying activity between target sites.

In another particular embodiment, the engineered SpCas9 protein is selected from one of the following group of variants K526Q-R661L-K855Q; R661L-K810Q-K855Q; R661L-K855Q-K1003L; and R661L-K855Q-R1060L (with reference to the numbering system of *Streptococcus pyogenes* Cas9, SpCas9). Thus, for example, the engineered SpCas9 protein variant could be K526Q-R661L-K855Q. Alternatively, for example, the engineered SpCas9 protein variant could be R661L-K810Q-K855Q. Alternatively, for example, the engineered SpCas9 protein variant could be R661L-K855Q-K1003L. Alternatively, for example, the engineered SpCas9 protein variant could be R661L-K855Q-R1060L. Members of this group of variants are similar to eSpCas9 1.1 in both specificity and activity levels; however, they are different from eSpCas9 1.1 in the mutation profiles.

In addition to the various mutations discussed above, the Cas9 protein can also be engineered by one or more mutations and/or deletions to inactivate one or both of the nuclease domains. Inactivation of one nuclease domain generates a Cas9 protein that cleaves one strand of a double-stranded sequence (*i.e*., a Cas9 nickase). The RuvC domain can be inactivated by mutations such as D10A, D8A, E762A, and/or D986A, and the HNH domain can be inactivated by mutations such as H840A, H559A, N854A, N856A, and/or N863A (with reference to the numbering system of *Streptococcus pyogenes* Cas9, SpCas9). Inactivation of both nuclease domains generates a Cas9 protein having no cleavage activity (*i.e*., a catalytically inactive or dead Cas9).

Further to the various mutations discussed above, the Cas9 protein can also be engineered by one or more amino acid substitutions, deletions, and/or insertions to have improved targeting specificity, improved fidelity, altered PAM specificity, decreased off-target effects, and/or increased stability. Non-limiting examples of one or more mutations that improve targeting specificity, improve fidelity, and/or decrease off-target effects include N497A, R661A, Q695A, K810A, K848A, K855A, Q926A, K1003A, R1060A, and/or D1135E (with reference to the numbering system of *Streptococcus pyogenes* Cas9, SpCas9).

In addition to the modifications discussed above, the Cas9 protein can also be engineered to comprise at least one heterologous domain, *i.e*., Cas9 is fused to one or more heterologous domains. In situations in which two or more heterologous domains are fused with Cas9, the two or more heterologous domains can be the same or they can be different. The one or more heterologous domains can be fused to the N terminal end, the C terminal end, an internal location, or combination thereof. The fusion can be direct via a chemical bond, or the linkage can be indirect via one or more linkers. In various embodiments, the heterologous domain is selected from a nuclear localization signal, a cell-penetrating domain, a marker or reporter domain which facilitates detection (fluorescent or enzymatic reporter proteins), a chromatin modification domain, an epigenetic modification domain (*e.g*., a cytidine deaminase domain, a histone acetyltransferase domain, and the like), a transcriptional regulation domain, a DNA or RNA deaminase domain, a uracil-DNA-glycosylase domain, a reverse transcriptase domain, a recombinase domain, an RNA aptamer binding domain, or a non-Cas9 nuclease domain.

### (a) Nuclear Localization Signals

In some embodiments the one or more heterologous domains can be a nuclear localization signal (NLS). Non-limiting examples of nuclear localization signals include PKKKRKV (SEQ ID NO:2), PKKKRRV (SEQ ID NO: 3), KRPAATKKAGQAKKKK (SEQ ID NO: 4), YGRKKRRQRRR (SEQ ID NO: 5), RKKRRQRRR (SEQ ID NO: 6), PAAKRVKLD (SEQ ID NO: 7), RQRRNELKRSP (SEQ ID NO: 8), VSRKRPRP (SEQ ID NO: 9), PPKKARED (SEQ ID NO: 10), PQPKKKPL (SEQ ID NO: 11), SALIKKKKKMAP (SEQ ID NO: 12), PKQKKRK (SEQ ID NO: 13), RKLKKKIKKL (SEQ ID NO: 14), REKKKFLKRR (SEQ ID NO: 15), KRKGDEVDGVDEVAKKKSKK (SEQ ID NO: 16), RKCLQAGMNLEARKTKK (SEQ ID NO: 17), NQSSNFGPMKGGNFGGRSSGPYGGGGQYFAKPRNQGGY (SEQ ID NO: 18), and RMRIZFKNKGKDTAELRRRRVEVSVELRKAKKDEQILKRRNV (SEQ ID NO: 19).

### (b) Cell-Penetrating Domains

In other embodiments, the one or more heterologous domains can be a cell-penetrating domain. Examples of suitable cell-penetrating domains include, without limit, GRKKRRQRRRPPQPKKKRKV (SEQ ID NO: 20), PLSSIFSRIGDPPKKKRKV (SEQ ID NO: 21), GALFLGWLGAAGSTMGAPKKKRKV (SEQ ID NO: 22), GALFLGFLGAAGSTMGAWSQPKKKRKV (SEQ ID NO: 23), KETWWETWWTEWSQPKKKRKV (SEQ ID NO: 24), YARAAARQARA (SEQ ID NO: 25), THRLPRRRRRR (SEQ ID NO: 26), GGRRARRRRRR (SEQ ID NO: 27), RRQRRTSKLMKR (SEQ ID NO: 28), GWTLNSAGYLLGKINLKALAALAKKIL (SEQ ID NO: 29), KALAWEAKLAKALAKALAKHLAKALAKALKCEA (SEQ ID NO: 30), and RQIKIWFQNRRMKWKK (SEQ ID NO: 31).

### (c) Marker Domains

In alternate embodiments, the one or more heterologous domains can be a marker domain. Marker domains include fluorescent proteins and purification or epitope tags. Suitable fluorescent proteins include, without limit, green fluorescent proteins (*e.g*., GFP, eGFP, GFP-2, tagGFP, turboGFP, Emerald, Azami Green, Monomeric Azami Green, CopGFP, AceGFP, ZsGreen1), yellow fluorescent proteins (*e.g*., YFP, EYFP, Citrine, Venus, YPet, PhiYFP, ZsYellow1), blue fluorescent proteins (*e.g*., BFP, EBFP, EBFP2, Azurite, mKalama1, GFPuv, Sapphire, T-sapphire), cyan fluorescent proteins (*e.g*., ECFP, Cerulean, CyPet, AmCyan1, Midoriishi-Cyan), red fluorescent proteins (*e.g*., mKate, mKate2, mPlum, DsRed monomer, mCherry, mRFP1, DsRed-Express, DsRed2, DsRed-Monomer, HcRed-Tandem, HcRed1, AsRed2, eqFP611, mRasberry, mStrawberry, Jred), orange fluorescent proteins (*e.g*., mOrange, mKO, Kusabira-Orange, Monomeric Kusabira-Orange, mTangerine, tdTomato), or combinations thereof. The marker domain can comprise tandem repeats of one or more fluorescent proteins (*e.g*., Suntag). Non-limiting examples of suitable purification or epitope tags include 6xHis (SEQ ID NO: 32), FLAG^{®}, HA, GST, Myc, SAM, and the like. Non-limiting examples of heterologous fusions which facilitate detection or enrichment of CRISPR complexes include streptavidin (Kipriyanov et al., Human Antibodies, 1995, 6(3):93-101), avidin (Airenne et al., Biomolecular Engineering, 1999, 16(1-4):87-92), monomeric forms of avidin (Laitinen et al., Journal of Biological Chemistry, 2003, 278(6):4010-4014), peptide tags which facilitate biotinylation during recombinant production (Cull et al., Methods in Enzymology, 2000, 326:430-440).

### (d) Chromatin Modulating Motifs

In still other embodiments, the one or more heterologous domain can be a chromatin modulating motif (CMM). Non-limiting examples of CMMs include nucleosome interacting peptides derived from high mobility group (HMG) proteins (*e.g*., HMGB1, HMGB2, HMGB3, HMGN1, HMGN2, HMGN3a, HMGN3b, HMGN4, and HMGN5 proteins), the central globular domain of histone H1 variants (*e.g*., histone H1.0, H1.1, H1.2, H1.3, H1.4, H1.5, H1.6, H1.7, H1.8, H1.9, and H.1.10), or DNA binding domains of chromatin remodeling complexes (*e.g*., SWI/SNF (SWItch/Sucrose Non-Fermentable), ISWI (Imitation SWItch), CHD (Chromodomain-Helicase-DNA binding), Mi-2/NuRD (Nucleosome Remodeling and Deacetylase), INO80, SWR1, and RSC complexes. In other embodiments, CMMs also can be derived from topoisomerases, helicases, or viral proteins. The source of the CMM can and will vary. CMMs can be from humans, animals (*i.e*., vertebrates and invertebrates), plants, algae, or yeast. Non-limiting examples of specific CMMs are listed in Table B below. Persons of skill in the art can readily identify homologs in other species and/or the relevant fusion motif therein.

**Table B: Chromatin Modulating Motifs**

| **Protein** | **Accession No.** | **Fusion Motif** |
|---|---|---|
| Human HMGN1 | P05114 | Full length |
| Human HMGN2 | P05204 | Full length |
| Human HMGN3a | Q15651 | Full length |
| Human HMGN3b | Q15651-2 | Full length |
| Human HMGN4 | O00479 | Full length |
| Human HMGN5 | P82970 | Nucleosome binding motif |
| Human HMGB1 | P09429 | Box A |
| Human histone H1.0 | P07305 | Globular motif |
| Human histone H1.2 | P16403 | Globular motif |
| Human CHD1 | O14646 | DNA binding motif |
| Yeast CHD1 | P32657 | DNA binding motif |
| Yeast ISWI | P38144 | DNA binding motif |
| Human TOP1 | P11387 | DNA binding motif |
| Human herpesvirus 8 LANA | J9QSF0 | Nucleosome binding motif |
| Human CMV IE1 | P13202 | Chromatin tethering motif |
| M. leprae DNA helicase | P40832 | HhH binding motif |

### (e) Epigenetic Modification Domains

In yet other embodiments, the one or more heterologous domains can be an epigenetic modification domain. Non-limiting examples of suitable epigenetic modification domains include those with DNA deamination (*e.g*., cytidine deaminase, adenosine deaminase, guanine deaminase), DNA methyltransferase activity (*e.g*., cytosine methyltransferase), DNA demethylase activity, DNA amination, DNA oxidation activity, DNA helicase activity, histone acetyltransferase (HAT) activity (*e.g*., HAT domain derived from E1A binding protein p300), histone deacetylase activity, histone methyltransferase activity, histone demethylase activity, histone kinase activity, histone phosphatase activity, histone ubiquitin ligase activity, histone deubiquitinating activity, histone adenylation activity, histone deadenylation activity, histone SUMOylating activity, histone deSUMOylating activity, histone ribosylation activity, histone deribosylation activity, histone myristoylation activity, histone demyristoylation activity, histone citrullination activity, histone alkylation activity, histone dealkylation activity, or histone oxidation activity. In specific embodiments, the epigenetic modification domain can comprise cytidine deaminase activity, adenosine deaminase activity, histone acetyltransferase activity, or DNA methyltransferase activity.

### (f) Transcriptional Regulation Domains

In other embodiments, the one or more heterologous domains can be a transcriptional regulation domain (*i.e*., a transcriptional activation domain or transcriptional repressor domain). Suitable transcriptional activation domains include, without limit, herpes simplex virus VP16 domain, VP64 (*i.e*., four tandem copies of VP16), VP160 (*i.e*., ten tandem copies of VP16), NFκB p65 activation domain (p65) , Epstein-Barr virus R transactivator (Rta) domain, VPR (*i.e*., VP64+p65+Rta), p300-dependent transcriptional activation domains, p53 activation domains 1 and 2, heat-shock factor 1 (HSF1) activation domains, Smad4 activation domains (SAD), cAMP response element binding protein (CREB) activation domains, E2A activation domains, nuclear factor of activated T-cells (NFAT) activation domains, or combinations thereof. Non-limiting examples of suitable transcriptional repressor domains include Kruppel-associated box (KRAB) repressor domains, Mxi repressor domains, inducible cAMP early repressor (ICER) domains, YY1 glycine rich repressor domains, Sp1-like repressors, E(spl) repressors, IκB repressors, Sin3 repressors, methyl-CpG binding protein 2 (MeCP2) repressors, or combinations thereof. Transcriptional activation or transcriptional repressor domains can be genetically fused to the Cas9 protein or bound via noncovalent protein-protein, protein-RNA, or protein-DNA interactions.

### (g) RNA Aptamer Binding Domains

In further embodiments, the one or more heterologous domains can be an RNA aptamer binding domain (Konermann et al., Nature, 2015, 517(7536):583-588; Zalatan et al., Cell, 2015, 160(1-2):339-50). Examples of suitable RNA aptamer protein domains include MS2 coat protein (MCP), PP7 bacteriophage coat protein (PCP), Mu bacteriophage Com protein, lambda bacteriophage N22 protein, stem-loop binding protein (SLBP), Fragile X mental retardation syndrome-related protein 1 (FXR1), proteins derived from bacteriophage such as AP205, BZ13, f1, f2, fd, fr, ID2, JP34/GA, JP501, JP34, JP500, KU1, M11, M12, MX1, NL95, PP7, φCb5, φCb8r, φCb12r, φCb23r, Qβ, R17, SP-β, TW18, TW19, and VK, fragments thereof, or derivatives thereof.

### (h) Non-Cas9 Nuclease Domains

In yet other embodiments, the one or more heterologous domains can be a non-Cas9 nuclease domain. Suitable nuclease domains can be obtained from any endonuclease or exonuclease. Non-limiting examples of endonucleases from which a nuclease domain can be derived include, but are not limited to, restriction endonucleases and homing endonucleases. In some embodiments, the nuclease domain can be derived from a type II-S restriction endonuclease. Type II-S endonucleases cleave DNA at sites that are typically several base pairs away from the recognition/binding site and, as such, have separable binding and cleavage domains. These enzymes generally are monomers that transiently associate to form dimers to cleave each strand of DNA at staggered locations. Non-limiting examples of suitable type II-S endonucleases include Bfil, Bpml, Bsal, Bsgl, BsmBI, Bsml, BspMI, Fokl, Mboll, and Sapl. In some embodiments, the nuclease domain can be a Fokl nuclease domain or a derivative thereof. The type II-S nuclease domain can be modified to facilitate dimerization of two different nuclease domains. For example, the cleavage domain of Fokl can be modified by mutating certain amino acid residues. By way of non-limiting example, amino acid residues at positions 446, 447, 479, 483, 484, 486, 487, 490, 491, 496, 498, 499, 500, 531, 534, 537, and 538 of Fokl nuclease domains are targets for modification. In specific embodiments, the Fokl nuclease domain can comprise a first Fokl half-domain comprising Q486E, I499L, and/or N496D mutations, and a second Fokl half-domain comprising E490K, I538K, and/or H537R mutations.

### (i) Nucleobase Modifying Enzymes

The engineered Cas9 variants described herein may also comprise a nucleobase modifying enzyme or catalytic domain thereof.

**A** variety of nucleobase modifying enzymes are suitable for use on the systems disclosed herein. The nucleobase modifying enzyme can be a DNA base editor. In some embodiments, the DNA base editor can be a cytidine deaminase, which converts cytidine into uridine, which is read by polymerase enzymes as thymine. Non-limiting examples of cytidine deaminases include cytidine deaminase 1 (CDA1), cytidine deaminase 2 (CDA2), activation-induced cytidine deaminase (AICDA), apolipoprotein B mRNA-editing complex (APOBEC) family cytidine deaminase (*e.g*., APOBEC1, APOBEC2, APOBEC3A, APOBEC3B, APOBEC3C, APOBEC3D/E, APOBEC3F, APOBEC3G, APOBEC3H, APOBEC4), APOBEC1 complementation factor/APOBEC1 stimulating factor (ACF1/ASF) cytidine deaminase, cytosine deaminase acting on RNA (CDAR), bacterial long isoform cytidine deaminase (CDD_{L}), and cytosine deaminase acting on tRNA (CDAT). In other embodiments, the DNA base editor can be an adenosine deaminase, which converts adenosine into inosine, which is read by polymerase enzymes as guanosine. Non-limiting examples of adenosine deaminases include tRNA adenine deaminase, adenosine deaminase, adenosine deaminase acting on RNA (ADAR), and adenosine deaminase acting on tRNA (ADAT).

The nucleobase modifying enzyme (base editor) can be wild type or a fragment thereof, a modified version thereof (*e.g*., non-essential domains can be deleted), or an engineered version thereof. The nucleobase modifying enzyme (base editor) can be of eukaryotic, bacterial, or archael origin.

In some embodiments, the nucleobase modifying enzyme (base editor) can be a cytidine deaminase or catalytic domain thereof. The cytidine deaminase can be of human, mouse, lamprey, abalone, or *E*. *coli* origin. In embodiments in which the nucleobase modifying enzyme is a cytidine deaminase, the RNA-guided nucleobase modifying system can further comprise at least one uracil glycosylase inhibitor (UGI) domain. Removal of uracil from DNA, which is the result of cytosine deamination, is inhibited by UGI. Suitable UGI domains are known in the art.

In some embodiments, a system that employs a cytidine deaminase and a UGI may have negative effects if these components are overexpressed. To prevent overexpression, a degradation tag may be added. Degradation tags signal a protein to be degraded by the protein recycling system. These degradation tags result in different protein half-lives. Non-limiting degradation tag examples are LVA, AAV, ASV and LAA.

### (j) Reverse Transcriptase

In some embodiments, the domain fused to the engineered SpCas9 variants described herein is a reverse transcriptase. Examples of the reverse transcriptases include avian myeloblastosis virus (AMV) reverse transcriptase and Moloney murine leukemia virus (MMLV) reverse transcriptase.

### (k) Recombinase / Integrase

In some embodiments, the domain fused to the engineered SpCas9 variants described herein is a recombinase or integrase. Non-limiting examples of suitable recombinases include the Cre recombinase, FLP recombinase, Gin recombinase, Bacteroides intN2 tyrosine integrase (coded by NBU2 gene), Streptomyces phage phiC31 (ϕC31) recombinase, coliphage P4 recombinase, coliphage lambda integrase, Listeria A118 phage recombinase, lentiviral or HIV integrase, and actinophage R4 Sre recombinase. Recombinases/integrases mediate recombination between two sequence specific recognition (or attachment) sites (*e.g*., an attP site and an attB site or two Cre/loxP sites) or can randomly insert DNA as with HIV integrase.

### (I) Linkers

The one or more heterologous domains can be linked directly to the Cas9 protein via one or more chemical bonds (*e.g*., covalent bonds), or the one or more heterologous domains can be linked indirectly to the Cas9 protein via one or more linkers.

A linker is a chemical group that connects one or more other chemical groups via at least one covalent bond. Suitable linkers include amino acids, peptides, nucleotides, nucleic acids, organic linker molecules (*e.g*., maleimide derivatives, N-ethoxybenzylimidazole, biphenyl-3,4',5-tricarboxylic acid, p-aminobenzyloxycarbonyl, and the like), disulfide linkers, and polymer linkers (*e.g*., PEG). The linker can include one or more spacing groups including, but not limited to alkylene, alkenylene, alkynylene, alkyl, alkenyl, alkynyl, alkoxy, aryl, heteroaryl, aralkyl, aralkenyl, aralkynyl and the like. The linker can be neutral, or carry a positive or negative charge. Additionally, the linker can be cleavable such that the linker's covalent bond that connects the linker to another chemical group can be broken or cleaved under certain conditions, including pH, temperature, salt concentration, light, a catalyst, or an enzyme. In some embodiments, the linker can be a peptide linker. The peptide linker can be a flexible amino acid linker (*e.g*., comprising small, non-polar or polar amino acids). Non-limiting examples of flexible linkers include LEGGGS (SEQ ID NO: 33), TGSG (SEQ ID NO: 34), GGSGGGSG (SEQ ID NO: 35), (GGGGS)₁₋₄ (SEQ ID NO: 36), and (Gly)₆₋₈ (SEQ ID NO: 37). Alternatively, the peptide linker can be a rigid amino acid linker. Such linkers include (EAAAK)₁₋₄ (SEQ ID NO: 38), A(EAAAK)₂₋₅A (SEQ ID NO: 39), PAPAP (SEQ ID NO: 40), and (AP)₆₋₈ (SEQ ID NO: 41). Additional examples of suitable linkers are well known in the art and programs to design linkers are readily available (*e.g*., Crasto et al., Protein Eng., 2000, 13(5):309-312).

### (m) Production of Engineered Cas9 Proteins

In some embodiments, the engineered Cas9 proteins can be produced recombinantly in cell-free systems, bacterial cells, or eukaryotic cells and purified using conventional purification methods. In other embodiments, the engineered Cas9 proteins are produced *in vivo* in eukaryotic cells of interest from nucleic acids encoding the engineered Cas9 proteins (see section (III) below and incorporated by reference in this section (I)).

In embodiments in which the engineered Cas9 protein comprises nuclease or nickase activity, the engineered Cas9 protein can further comprise at least one nuclear localization signal, cell-penetrating domain, and/or marker domain, as well as at least one chromatin disrupting domain. In embodiments in which the engineered Cas9 protein is linked to an epigenetic modification domain, the engineered Cas9 protein can further comprise at least one nuclear localization signal, cell-penetrating domain, and/or marker domain, as well as at least one chromatin disrupting domain. Furthermore, in embodiments in which the engineered Cas9 protein is linked to a transcriptional regulation domain, the engineered Cas9 protein can further comprise at least one nuclear localization signal, cell-penetrating domain, and/or marker domain, as well as at least one chromatin disrupting domain and/or at least one RNA aptamer binding domain.

### (II) Engineered Cas9 Systems

Another aspect of the present disclosure provides engineered Cas9 systems comprising engineered Cas9 protein variants as discussed above in section (I) incorporated by reference in this section **(II)** (for example, an engineered Cas9 protein variant including the mutations selected from the group defined in claim 1 (with reference to the numbering system of *Streptococcus pyogenes* Cas9, SpCas9) and engineered guide RNAs, wherein each engineered guide RNA is designed to complex with a specific engineered Cas9 protein. Each engineered guide RNA comprises a 5' guide sequence designed to hybridize with a target sequence in a double-stranded sequence, wherein the target sequence is 5' to a protospacer adjacent motif (PAM).

### (a) Engineered guide RNAs

The engineered guide RNA is designed to complex with a specific engineered Cas9 protein. A guide RNA comprises (i) a CRISPR RNA (crRNA) that contains a guide sequence at the 5' end that hybridizes with a target sequence and (ii) a transacting crRNA (tracrRNA) sequence that recruits the Cas9 protein. The crRNA guide sequence of each guide RNA is different (*i.e*., is sequence specific). The tracrRNA sequence is generally the same in guide RNAs designed to complex with a Cas9 protein from a particular bacterial species.

The crRNA guide sequence is designed to hybridize with a target sequence (*i.e*., protospacer) in a double-stranded sequence. In general, the complementarity between the crRNA and the target sequence is at least 80%, at least 85%, at least 90%, at least 95%, or at least 99%. In specific embodiments, the complementarity is complete (*i.e*., 100%). In various embodiments, the length of the crRNA guide sequence can range from about 15 nucleotides to about 25 nucleotides. For example, the crRNA guide sequence can be about 15, 16, 17, 18, 19, 20, 21, 22, 23, 24, or 25 nucleotides in length. In specific embodiments, the crRNA is about 19, 20, or 21 nucleotides in length. In one embodiment, the crRNA guide sequence has a length of 20 nucleotides.

**The** guide RNA comprises repeat sequence that forms at least one stem loop structure, which interacts with the Cas9 protein, and 3' sequence that remains single-stranded. The length of each loop and stem can vary. For example, the loop can range from about 3 to about 10 nucleotides in length, and the stem can range from about 6 to about 20 base pairs in length. The stem can comprise one or more bulges of 1 to about 10 nucleotides. The length of the single-stranded 3' region can vary. The tracrRNA sequence in the engineered guide RNA generally is based upon the coding sequence of wild type tracrRNA in the bacterial species of interest. The wild-type sequence can be modified to facilitate secondary structure formation, increased secondary structure stability, facilitate expression in eukaryotic cells, and so forth. For example, one or more nucleotide changes can be introduced into the guide RNA coding sequence (see Example 3, below). The tracrRNA sequence can range in length from about 50 nucleotides to about 300 nucleotides. In various embodiments, the tracrRNA can range in length from about 50 to about 90 nucleotides, from about 90 to about 110 nucleotides, from about 110 to about 130 nucleotides, from about 130 to about 150 nucleotides, from about 150 to about 170 nucleotides, from about 170 to about 200 nucleotides, from about 200 to about 250 nucleotides, or from about 250 to about 300 nucleotides.

In general, the engineered guide RNA is a single molecule (*i.e*., a chimeric single guide RNA or sgRNA), wherein the crRNA sequence is linked to the tracrRNA sequence. In some embodiments, however, the engineered guide RNA can be two separate molecules (*e.g*., a dual molecule guide RNA). For example, the guide RNA may include a first molecule (or region) comprising the crRNA that contains 3' sequence (comprising from about 6 to about 20 nucleotides) that is capable of base pairing with the 5' end of a second molecule, and a second molecule (or region) comprising the tracrRNA that contains 5' sequence (comprising from about 6 to about 20 nucleotides) that is capable of base pairing with the 3' end of the first molecule (or region).

In some embodiments, the tracrRNA sequence of the engineered guide RNA can be modified to comprise one or more aptamer sequences (Konermann et al., Nature, 2015, 517(7536):583-588; Zalatan et al., Cell, 2015, 160(1-2):339-50). Suitable aptamer sequences include those that bind adaptor proteins chosen from MCP, PCP, Com, SLBP, FXR1, AP205, BZ13, f1, f2, fd, fr, ID2, JP34/GA, JP501, JP34, JP500, KU1, M11, M12, MX1, NL95, PP7, φCb5, φCb8r, φCb12r, φCb23r, Qβ, R17, SP-β, TW18, TW19, VK, fragments thereof, or derivatives thereof. Those of skill in the art appreciate that the length of the aptamer sequence can vary.

In other embodiments, the guide RNA can further comprise at least one detectable label. The detectable label can be a fluorophore (*e.g*., FAM, TMR, Cy3, Cy5, Texas Red, Oregon Green, Alexa Fluors, Halo tags, or suitable fluorescent dye), a detection tag (e.g., biotin, digoxigenin, and the like), quantum dots, or gold particles.

The guide RNA can comprise standard ribonucleotides and/or modified ribonucleotides. In some embodiment, the guide RNA can comprise standard or modified deoxyribonucleotides. In embodiments in which the guide RNA is enzymatically synthesized (*i.e*., *in vivo* or *in vitro*), the guide RNA generally comprises standard ribonucleotides. In embodiments in which the guide RNA is chemically synthesized, the guide RNA can comprise standard or modified ribonucleotides and/or deoxyribonucleotides. Modified ribonucleotides and/or deoxyribonucleotides include base modifications (*e.g*., pseudouridine, 2-thiouridine, N6-methyladenosine, and the like) and/or sugar modifications (*e.g*., 2'-O-methy, 2'-fluoro, 2'-amino, locked nucleic acid (LNA), and so forth). The backbone of the guide RNA can also be modified to comprise phosphorothioate linkages, boranophosphate linkages, or peptide nucleic acids.

### (b) PAM Sequence

The engineered Cas9 systems detailed above target specific sequences in double-stranded DNA that are located upstream of PAM sequences. The PAM sequences may include a canonical 5'-NGG-3' PAM or a non-canonical PAM, such as a 5'-NAG-3' PAM. In some embodiment, the engineered Cas9 systems detailed above may be modified to recognize alternative PAMs, such as 5'-NGAN-3', 5'-NGNG-3', and 5'-NGCG-3' PAMs.

### (III) Nucleic Acids

A further aspect of the present disclosure provides nucleic acids encoding the engineered Cas9 protein variants and systems described above in sections (I) and **(II)** incorporated by reference in this section (III) (for example, an engineered Cas9 protein variant including the mutations selected from the group defined in claim 1 (with reference to the numbering system of *Streptococcus pyogenes* Cas9, SpCas9). The proteins and systems can be encoded by single nucleic acids or multiple nucleic acids. The nucleic acids can be DNA or RNA, linear or circular, single-stranded or double-stranded. The RNA or DNA can be codon optimized for efficient translation into protein in the eukaryotic cell of interest. Codon optimization programs are available as freeware or from commercial sources.

In some embodiments, the nucleic acid encoding the engineered Cas9 protein can be RNA. The RNA can be enzymatically synthesized *in vitro.* For this, DNA encoding the engineered Cas9 protein can be operably linked to a promoter sequence that is recognized by a phage RNA polymerase for *in vitro* RNA synthesis. For example, the promoter sequence can be a T7, T3, or SP6 promoter sequence or a variation of a T7, T3, or SP6 promoter sequence. The DNA encoding the engineered protein can be part of a vector, as detailed below. In such embodiments, the *in vitro-*transcribed RNA can be purified, capped, and/or polyadenylated. In other embodiments, the RNA encoding the engineered Cas9 protein can be part of a self-replicating RNA (Yoshioka et al., Cell Stem Cell, 2013, 13:246-254). The self-replicating RNA can be derived from a noninfectious, self-replicating Venezuelan equine encephalitis (VEE) virus RNA replicon, which is a positive-sense, single-stranded RNA that is capable of self-replicating for a limited number of cell divisions, and which can be modified to code proteins of interest (Yoshioka et al., Cell Stem Cell, 2013, 13:246-254).

In other embodiments, the nucleic acid encoding the engineered Cas9 protein can be DNA. The DNA coding sequence can be operably linked to at least one promoter control sequence for expression in the cell of interest. In certain embodiments, the DNA coding sequence can be operably linked to a promoter sequence for expression of the engineered Cas9 protein in bacterial (*e.g.*, *E. coli*) cells or eukaryotic (*e.g*., yeast, insect, or mammalian) cells. Suitable bacterial promoters include, without limit, T7 promoters, *lac* operon promoters, *trp* promoters, *tac* promoters (which are hybrids of *trp* and *lac* promoters), variations of any of the foregoing, and combinations of any of the foregoing. Non-limiting examples of suitable eukaryotic promoters include constitutive, regulated, or cell- or tissue-specific promoters. Suitable eukaryotic constitutive promoter control sequences include, but are not limited to, cytomegalovirus immediate early promoter (CMV), simian virus (SV40) promoter, adenovirus major late promoter, Rous sarcoma virus (RSV) promoter, mouse mammary tumor virus (MMTV) promoter, phosphoglycerate kinase (PGK) promoter, elongation factor (ED1)-alpha promoter, ubiquitin promoters, actin promoters, tubulin promoters, immunoglobulin promoters, fragments thereof, or combinations of any of the foregoing. Examples of suitable eukaryotic regulated promoter control sequences include without limit those regulated by heat shock, metals, steroids, antibiotics, or alcohol. Non-limiting examples of tissue-specific promoters include B29 promoter, CD14 promoter, CD43 promoter, CD45 promoter, CD68 promoter, desmin promoter, elastase-1 promoter, endoglin promoter, fibronectin promoter, Flt-1 promoter, GFAP promoter, GPIIb promoter, ICAM-2 promoter, INF-β promoter, Mb promoter, Nphsl promoter, OG-2 promoter, SP-B promoter, SYN1 promoter, and WASP promoter. The promoter sequence can be wild-type or it can be modified for more efficient or efficacious expression. In some embodiments, the DNA coding sequence also can be linked to a polyadenylation signal (*e.g*., SV40 polyA signal, bovine growth hormone (BGH) polyA signal, *etc.*) and/or at least one transcriptional termination sequence. In some situations, the engineered Cas9 protein can be purified from the bacterial or eukaryotic cells.

In still other embodiments, the engineered guide RNA can be encoded by DNA. In some instances, the DNA encoding the engineered guide RNA can be operably linked to a promoter sequence that is recognized by a phage RNA polymerase for *in vitro* RNA synthesis. For example, the promoter sequence can be a T7, T3, or SP6 promoter sequence or a variation of a T7, T3, or SP6 promoter sequence. In other instances, the DNA encoding the engineered guide RNA can be operably linked to a promoter sequence that is recognized by RNA polymerase III (Pol III) for expression in eukaryotic cells of interest. Examples of suitable Pol III promoters include, but are not limited to, mammalian U6, U3, H1, and 7SL RNA promoters.

In various embodiments, the nucleic acid encoding the engineered Cas9 protein can be present in a vector. In some embodiments, the vector can further comprise nucleic acid encoding the engineered guide RNA. Suitable vectors include plasmid vectors, viral vectors, and self-replicating RNA (Yoshioka et al., Cell Stem Cell, 2013, 13:246-254). In some embodiments, the nucleic acid encoding the complex or fusion protein can be present in a plasmid vector. Non-limiting examples of suitable plasmid vectors include pUC, pBR322, pET, pBluescript, and variants thereof. In other embodiments, the nucleic acid encoding the complex or fusion protein can be part of a viral vector (*e.g*., lentiviral vectors, adeno-associated viral vectors, adenoviral vectors, and so forth). The plasmid or viral vector can comprise additional expression control sequences (*e.g*., enhancer sequences, Kozak sequences, polyadenylation sequences, transcriptional termination sequences, *etc.*), selectable marker sequences (*e.g*., antibiotic resistance genes), origins of replication, and the like. Additional information about vectors and use thereof can be found in "Current Protocols in Molecular Biology" Ausubel et al., John Wiley & Sons, New York, 2003 or "Molecular Cloning: A Laboratory Manual" Sambrook & Russell, Cold Spring Harbor Press, Cold Spring Harbor, NY, 3rd edition, 2001.

### (IV) Eukaryotic Cells

Another aspect of the present disclosure comprises eukaryotic cells comprising at least one engineered Cas9 protein variants as detailed above in section (I) incorporated by reference in this section (IV) (for example, an engineered Cas9 protein variant including the mutations selected from the group defined in claim 1 (with reference to the numbering system of *Streptococcus pyogenes* Cas9, SpCas9) and/or at least one nucleic acid encoding an engineered Cas9 protein and/or system and/or engineered guide RNA as detailed above in sections (I), (II) and (III) (each of which is incorporated by reference in this section (IV)).

The eukaryotic cell can be a human cell, a non-human mammalian cell, a non-mammalian vertebrate cell, an invertebrate cell, a plant cell, or a single cell eukaryotic organism. Examples of suitable eukaryotic cells are detailed below in section (V)(c). The eukaryotic cell can be in vitro or ex vivo.

### (V) Methods for Modifying Sequences

A further aspect of the present disclosure encompasses methods for modifying a chromosomal sequence in eukaryotic cells, wherein the methods are not a process for modifying the germ line genetic identity of human beings and are not a method for treatment of the human or animal body by surgery or therapy. In general, the methods comprise introducing into the eukaryotic cell of interest at least one engineered Cas9 system as detailed above in section (II), which further includes an engineered Cas9 protein variant as detailed above in section (I) each of which sections (I) and (II) are incorporated by reference in this section (V) (for example, an engineered Cas9 protein variant including the mutations selected from the group defined in claim 1 (with reference to the numbering system of *Streptococcus pyogenes* Cas9, SpCas9), and/or at least one nucleic acid encoding an engineered Cas9 protein and/or system and/or engineered guide RNA as detailed above in sections (I), (II) and (III) (each of which is incorporated by reference in this section (V)).

In embodiments in which the engineered Cas9 protein comprises nuclease or nickase activity, the chromosomal sequence modification can comprise a substitution of at least one nucleotide, a deletion of at least one nucleotide, an insertion of at least one nucleotide. In some iterations, the method comprises introducing into the eukaryotic cell one engineered Cas9 system comprising nuclease activity or two engineered Cas9 systems comprising nickase activity and no donor polynucleotide, such that the engineered Cas9 system or systems introduce a double-stranded break in the target site in the chromosomal sequence and repair of the double-stranded break by cellular DNA repair processes introduces at least one nucleotide change (*i.e*., indel), thereby inactivating the chromosomal sequence (*i.e*., gene knock-out). In other iterations, the method comprises introducing into the eukaryotic cell one engineered Cas9 system comprising nuclease activity or two engineered Cas9 systems comprising nickase activity, as well as the donor polynucleotide, such that the engineered Cas9 system or systems introduce a double-stranded break in the target site in the chromosomal sequence and repair of the double-stranded break by cellular DNA repair processes leads to insertion or exchange of sequence in the donor polynucleotide into the target site in the chromosomal sequence (i.e., gene correction or gene knock-in).

In embodiments, in which the engineered Cas9 protein comprises epigenetic modification activity or transcriptional regulation activity, the chromosomal sequence modification can comprise a conversion of at least one nucleotide in or near the target site, a modification of at least one nucleotide in or near the target site, a modification of at least one histone protein in or near the target site, and/or a change in transcription in or near the target site in the chromosomal sequence.

Still further, it will be understood that the engineered Cas9 variants described herein may also be used to modify other than eukaryotic cells, such as microbial genomes.

### (a) Introduction into the Cell

As mentioned above, the method comprises introducing into the eukaryotic cell at least one engineered Cas9 system and/or nucleic acid encoding said system (and optional donor polynucleotide). The at least one system and/or nucleic acid/donor polynucleotide can be introduced into the cell of interest by a variety of means.

In some embodiments, the cell can be transfected with the appropriate molecules (*i.e*., protein, DNA, and/or RNA). Suitable transfection methods include nucleofection (or electroporation), calcium phosphate-mediated transfection, cationic polymer transfection (*e.g*., DEAE-dextran or polyethylenimine), viral transduction, virosome transfection, virion transfection, liposome transfection, cationic liposome transfection, immunoliposome transfection, nonliposomal lipid transfection, dendrimer transfection, heat shock transfection, magnetofection, lipofection, gene gun delivery, impalefection, sonoporation, optical transfection, and proprietary agent-enhanced uptake of nucleic acids. Transfection methods are well known in the art (see, *e.g.*, "Current Protocols in Molecular Biology" Ausubel et al., John Wiley & Sons, New York, 2003 or "Molecular Cloning: A Laboratory Manual" Sambrook & Russell, Cold Spring Harbor Press, Cold Spring Harbor, NY, 3rd edition, 2001). In other embodiments, the molecules can be introduced into the cell by microinjection. For example, the molecules can be injected into the cytoplasm or nuclei of the cells of interest. The amount of each molecule introduced into the cell can vary, but those skilled in the art are familiar with means for determining the appropriate amount.

**The** various molecules can be introduced into the cell simultaneously or sequentially. For example, the engineered Cas9 system (or its encoding nucleic acid) and the donor polynucleotide can be introduced at the same time. Alternatively, one can be introduced first and then the other can be introduced later into the cell.

In general, the cell is maintained under conditions appropriate for cell growth and/or maintenance. Suitable cell culture conditions are well known in the art and are described, for example, in Santiago et al., Proc. Natl. Acad. Sci. USA, 2008, 105:5809-5814; Moehle et al. Proc. Natl. Acad. Sci. USA, 2007, 104:3055-3060; Urnov et al., Nature, 2005, 435:646-651; and Lombardo et al., Nat. Biotechnol., 2007, 25:1298-1306. Those of skill in the art appreciate that methods for culturing cells are known in the art and can and will vary depending on the cell type. Routine optimization may be used, in all cases, to determine the best techniques for a particular cell type.

### (b) Optional Donor Polynucleotide

In embodiments in which the engineered Cas9 protein comprises nuclease or nickase activity, the method can further comprise introducing at least one donor polynucleotide into the cell. The donor polynucleotide can be single-stranded or double-stranded, linear or circular, and/or RNA or DNA. In some embodiments, the donor polynucleotide can be a vector, *e.g*., a plasmid vector.

**The** donor polynucleotide comprises at least one donor sequence. In some aspects, the donor sequence of the donor polynucleotide can be a modified version of an endogenous or native chromosomal sequence. For example, the donor sequence can be essentially identical to a portion of the chromosomal sequence at or near the sequence targeted by the engineered Cas9 system, but which comprises at least one nucleotide change. Thus, upon integration or exchange with the native sequence, the sequence at the targeted chromosomal location comprises at least one nucleotide change. For example, the change can be an insertion of one or more nucleotides, a deletion of one or more nucleotides, a substitution of one or more nucleotides, or combinations thereof. As a consequence of the "gene correction" integration of the modified sequence, the cell can produce a modified gene product from the targeted chromosomal sequence.

In other aspects, the donor sequence of the donor polynucleotide can be an exogenous sequence. As used herein, an "exogenous" sequence refers to a sequence that is not native to the cell, or a sequence whose native location is in a different location in the genome of the cell. For example, the exogenous sequence can comprise protein coding sequence, which can be operably linked to an exogenous promoter control sequence such that, upon integration into the genome, the cell is able to express the protein coded by the integrated sequence. Alternatively, the exogenous sequence can be integrated into the chromosomal sequence such that its expression is regulated by an endogenous promoter control sequence. In other iterations, the exogenous sequence can be a transcriptional control sequence, another expression control sequence, an RNA coding sequence, and so forth. As noted above, integration of an exogenous sequence into a chromosomal sequence is termed a "knock in."

**As** can be appreciated by those skilled in the art, the length of the donor sequence can and will vary. For example, the donor sequence can vary in length from several nucleotides to hundreds of nucleotides to hundreds of thousands of nucleotides.

Typically, the donor sequence in the donor polynucleotide is flanked by an upstream sequence and a downstream sequence, which have substantial sequence identity to sequences located upstream and downstream, respectively, of the sequence targeted by the engineered Cas9 system. Because of these sequence similarities, the upstream and downstream sequences of the donor polynucleotide permit homologous recombination between the donor polynucleotide and the targeted chromosomal sequence such that the donor sequence can be integrated into (or exchanged with) the chromosomal sequence.

The upstream sequence, as used herein, refers to a nucleic acid sequence that shares substantial sequence identity with a chromosomal sequence upstream of the sequence targeted by the engineered Cas9 system. Similarly, the downstream sequence refers to a nucleic acid sequence that shares substantial sequence identity with a chromosomal sequence downstream of the sequence targeted by the engineered Cas9 system. As used herein, the phrase "substantial sequence identity" refers to sequences having at least about 75% sequence identity. Thus, the upstream and downstream sequences in the donor polynucleotide can have about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with sequence upstream or downstream to the target sequence. In an exemplary embodiment, the upstream and downstream sequences in the donor polynucleotide can have about 95% or 100% sequence identity with chromosomal sequences upstream or downstream to the sequence targeted by the engineered Cas9 system.

In some embodiments, the upstream sequence shares substantial sequence identity with a chromosomal sequence located immediately upstream of the sequence targeted by the engineered Cas9 system. In other embodiments, the upstream sequence shares substantial sequence identity with a chromosomal sequence that is located within about one hundred (100) nucleotides upstream from the target sequence. Thus, for example, the upstream sequence can share substantial sequence identity with a chromosomal sequence that is located about 1 to about 20, about 21 to about 40, about 41 to about 60, about 61 to about 80, or about 81 to about 100 nucleotides upstream from the target sequence. In some embodiments, the downstream sequence shares substantial sequence identity with a chromosomal sequence located immediately downstream of the sequence targeted by the engineered Cas9 system. In other embodiments, the downstream sequence shares substantial sequence identity with a chromosomal sequence that is located within about one hundred (100) nucleotides downstream from the target sequence. Thus, for example, the downstream sequence can share substantial sequence identity with a chromosomal sequence that is located about 1 to about 20, about 21 to about 40, about 41 to about 60, about 61 to about 80, or about 81 to about 100 nucleotides downstream from the target sequence.

Each upstream or downstream sequence can range in length from about 20 nucleotides to about 5000 nucleotides. In some embodiments, upstream and downstream sequences can comprise about 50, 100, 200, 300, 400, 500, 600, 700, 800, 900, 1000, 1100, 1200, 1300, 1400, 1500, 1600, 1700, 1800, 1900, 2000, 2100, 2200, 2300, 2400, 2500, 2600, 2800, 3000, 3200, 3400, 3600, 3800, 4000, 4200, 4400, 4600, 4800, or 5000 nucleotides. In specific embodiments, upstream and downstream sequences can range in length from about 50 to about 1500 nucleotides.

### (c) Cell Types

**A** variety of cells are suitable for use in the methods disclosed herein, including prokaryotic cells (*e.g*., bacterium) and eukaryotic cells (*e.g*., animal, insect, and plant cells). For example, the cell can be a human cell, a non-human mammalian cell, a non-mammalian vertebrate cell, an invertebrate cell, an insect cell, a plant cell, a yeast cell, or a single cell eukaryotic organism. In some embodiments, the cell can be a non-human one cell embryo. For example, a non-human mammalian embryo including rat, hamster, rodent, rabbit, feline, canine, ovine, porcine, bovine, equine, and primate embryos. In still other embodiments, the cell can be a stem cell such as embryonic stem cells, ES-like stem cells, fetal stem cells, adult stem cells, and the like. In one embodiment, the stem cell is not a human embryonic stem cell. Furthermore, the stem cells may include those made by the techniques disclosed in WO2003/046141, or Chung et al. (Cell Stem Cell, 2008, 2:113-117). The cell can be *in vitro* (*i.e.,* in culture), or ex vivo (*i.e*., within tissue isolated from an organism). In exemplary embodiments, the cell is a mammalian cell or mammalian cell line. In particular embodiments, the cell is a human cell or human cell line.

By way of example, in some embodiments, the eukaryotic cell, or a population of eukaryotic cells, is a T-cell, a CD8⁺ T-cell, a CD8⁺ naive T cell, a central memory T cell, an effector memory T-cell, a CD4⁺ T-cell, a stem cell memory T-cell, a helper T-cell, a regulatory T-cell, a cytotoxic T-cell, a natural killer T-cell, a hematopoietic stem cell, a long term hematopoietic stem cell, a short term hematopoietic stem cell, a multipotent progenitor cell, a lineage restricted progenitor cell, a lymphoid progenitor cell, a pancreatic progenitor cell, an endocrine progenitor cell, an exocrine progenitor cell, a myeloid progenitor cell, a common myeloid progenitor cell, an erythroid progenitor cell, a megakaryocyte erythroid progenitor cell, a monocytic precursor cell, an endocrine precursor cell, an exocrine cell, a fibroblast, a hepatoblast, a myoblast, a macrophage, an islet beta-cell, a cardiomyocyte, a blood cell, a ductal cell, an acinar cell, an alpha cell, a beta cell, a delta cell, a PP cell, a cholangiocyte, a retinal cell, a photoreceptor cell, a rod cell, a cone cell, a retinal pigmented epithelium cell, a trabecular meshwork cell, a cochlear hair cell, an outer hair cell, an inner hair cell, a pulmonary epithelial cell, a bronchial epithelial cell, an alveolar epithelial cell, a pulmonary epithelial progenitor cell, a striated muscle cell, a cardiac muscle cell, a muscle satellite cell, a myocyte, a neuron, a neuronal stem cell, a mesenchymal stem cell, an induced pluripotent stem (iPS) cell, an embryonic stem cell, a monocyte, a megakaryocyte, a neutrophil, an eosinophil, a basophil, a mast cell, a reticulocyte, a B cell, *e.g*. a progenitor B cell, a Pre B cell, a Pro B cell, a memory B cell, a plasma B cell, a gastrointestinal epithelial cell, a biliary epithelial cell, a pancreatic ductal epithelial cell, an intestinal stem cell, a hepatocyte, a liver stellate cell, a Kupffer cell, an osteoblast, an osteoclast, an adipocyte (*e.g*., a brown adipocyte, or a white adipocyte), a preadipocyte, a pancreatic precursor cell, a pancreatic islet cell, a pancreatic beta cell, a pancreatic alpha cell, a pancreatic delta cell, a pancreatic exocrine cell, a Schwann cell, or an oligodendrocyte, or a population of such cells. Non-limiting examples of suitable mammalian cells or cell lines include human induced pluripotent stem cells (hiPSC), human T cells (autologous or allogenic), human B cells, human macrophages, human hematopoietic stem cells, (hHSC), human liver cells, human retinal cells, pancreatic islets, human embryonic kidney cells (HEK293, HEK293T); human cervical carcinoma cells (HELA); human lung cells (W138); human liver cells (Hep G2); human U2-OS osteosarcoma cells, human A549 cells, human A-431 cells, and human K562 cells; Chinese hamster ovary (CHO) cells, baby hamster kidney (BHK) cells; mouse myeloma NS0 cells, mouse embryonic fibroblast 3T3 cells (NIH3T3), mouse B lymphoma A20 cells; mouse melanoma B16 cells; mouse myoblast C2C12 cells; mouse myeloma SP2/0 cells; mouse embryonic mesenchymal C3H-10T1/2 cells; mouse carcinoma CT26 cells, mouse prostate DuCuP cells; mouse breast EMT6 cells; mouse hepatoma Hepa1c1c7 cells; mouse myeloma J5582 cells; mouse epithelial MTD-1A cells; mouse myocardial MyEnd cells; mouse renal RenCa cells; mouse pancreatic RIN-5F cells; mouse melanoma X64 cells; mouse lymphoma YAC-1 cells; rat glioblastoma 9L cells; rat B lymphoma RBL cells; rat neuroblastoma B35 cells; rat hepatoma cells (HTC); buffalo rat liver BRL 3A cells; canine kidney cells (MDCK); canine mammary (CMT) cells; rat osteosarcoma D17 cells; rat monocyte/macrophage DH82 cells; monkey kidney SV-40 transformed fibroblast (COS7) cells; monkey kidney CVI-76 cells; African green monkey kidney (VERO-76) cells. An extensive list of mammalian cell lines may be found in the American Type Culture Collection catalog (ATCC, Manassas, VA).

Still other aspects of the disclosure include a plant engineered using a nucleic acid or a vector as described above or a plant temporarily or permanently modified by way of an engineered SpCas9 variant of the disclosure. For example, the plant can be a crop (*i.e*., rice, soybean, wheat, tobacco, cotton, alfalfa, canola, corn, sugar beet, and the like).

### (VI) Applications

The compositions and methods disclosed herein can be used in a variety of therapeutic, diagnostic, industrial, and research applications. In some embodiments, the present disclosure can be used to modify any chromosomal sequence of interest in a cell, animal, or plant in order to model and/or study the function of genes, study genetic or epigenetic conditions of interest, or study biochemical pathways involved in various diseases or disorders. For example, transgenic organisms can be created that model diseases or disorders, wherein the expression of one or more nucleic acid sequences associated with a disease or disorder is altered. The disease model can be used to study the effects of mutations on the organism, study the development and/or progression of the disease, study the effect of a pharmaceutically active compound on the disease, and/or assess the efficacy of a potential gene therapy strategy.

In other embodiments, the compositions and methods can be used to perform efficient and cost effective functional genomic screens, which can be used to study the function of genes involved in a particular biological process and how any alteration in gene expression can affect the biological process, or to perform saturating or deep scanning mutagenesis of genomic loci in conjunction with a cellular phenotype. Saturating or deep scanning mutagenesis can be used to determine critical minimal features and discrete vulnerabilities of functional elements required for gene expression, drug resistance, and reversal of disease, for example.

In further embodiments, the compositions and methods disclosed herein can be used for diagnostic tests to establish the presence of a disease or disorder and/or for use in determining treatment options. Examples of suitable diagnostic tests include detection of specific mutations in cancer cells (*e.g*., specific mutation in EGFR, HER2, and the like), detection of specific mutations associated with particular diseases (*e.g*., trinucleotide repeats, mutations in β-globin associated with sickle cell disease, specific SNPs, *etc.*), detection of hepatitis, detection of viruses (*e.g*., Zika), and so forth.

In additional embodiments, the compositions and methods disclosed herein can be used to correct genetic mutations associated with a particular disease or disorder such as, *e.g*., correct globin gene mutations associated with sickle cell disease or thalassemia, correct mutations in the adenosine deaminase gene associated with severe combined immune deficiency (SCID), reduce the expression of HTT, the disease-causing gene of Huntington's disease, or correct mutations in the rhodopsin gene for the treatment of retinitis pigmentosa. Such modifications may be made in cells *ex vivo.*

In still other embodiments, the compositions and methods disclosed herein can be used to generate crop plants with improved traits or increased resistance to environmental stresses. The present disclosure can also be used to generate farm animal with improved traits or production animals. For example, pigs have many features that make them attractive as biomedical models, especially in regenerative medicine or xenotransplantation.

By way of example, the present disclosure provides sequence of nucleotides or a nucleic acid or a vector, as described above, for use as a medicament for gene therapy. The present disclosure also provides a pharmaceutical composition comprising a sequence of nucleotides or a nucleic acid or a vector, as above described, and at least one pharmaceutically acceptable excipient. The present disclosure also provides a pharmaceutical composition comprising a recombinant Cas9 polypeptide containing the mutations described above and at least one pharmaceutically acceptable excipient. Pharmaceutically acceptable excipient(s) typically include inactive ingredients used as a vehicle (for example, water, capsule shell etc.), a diluent, or a component to constitute a dosage form or pharmaceutical composition comprising a drug such as a therapeutic agent. Pharmaceutically acceptable excipient(s) also encompasses a typically inactive ingredient that imparts cohesive function (*i.e*., binder), disintegrating function (*i.e*., disintegrator), lubricant function (lubricating agent), and/or the other function (*i.e*., solvent, surfactant etc.) to the composition. Further, the present disclosure provides in vitro use of a sequence of nucleotides or a nucleic acid or a vector, as above described, for genome engineering, cell engineering, protein expression or other biotechnology applications. Still further, the present disclosure provides the use in vitro of a recombinant Cas9 polypeptide containing the mutations described above together with a guide RNA (*e.g*., a single molecule (*i.e*., chimeric) guide RNA or a dual molecule (*i.e*., two-part) guide RNA for genome engineering, cell engineering, protein expression or other biotechnology applications.

Other aspects of the disclosure are directed to kits including various components described herein, such as the Cas9 protein variants described herein, guide RNAs, vectors, primers, and the like, including instructions for their use in genome engineering, cell engineering, protein expression or other biotechnology applications.

### DEFINITIONS

The following definitions and methods are provided to better define the present invention and to guide those of ordinary skill in the art in the practice of the present invention. Unless otherwise noted, terms are to be understood according to conventional usage by those of ordinary skill in the relevant art.

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd Ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them unless specified otherwise.

When introducing elements of the present disclosure or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

The term "about" when used in relation to a numerical value, x, for example means x ± 5%.

As used herein, the terms "complementary" or "complementarity" refer to the association of double-stranded nucleic acids by base pairing through specific hydrogen bonds. The base pairing may be standard Watson-Crick base pairing (*e.g*., 5'-A G T C-3' pairs with the complementary sequence 3'-T C A G-5'). The base pairing also may be Hoogsteen or reversed Hoogsteen hydrogen bonding. Complementarity is typically measured with respect to a duplex region and thus, excludes overhangs, for example. Complementarity between two strands of the duplex region may be partial and expressed as a percentage (*e.g*., 70%), if only some (*e.g*., 70%) of the bases are complementary. The bases that are not complementary are "mismatched." Complementarity may also be complete (*i.e*., 100%), if all the bases in the duplex region are complementary.

As used herein, the term "CRISPR/Cas system" or "Cas9 system" refers to a complex comprising a Cas9 protein (*i.e*., nuclease, nickase, or catalytically dead protein) and a guide RNA.

The term "endogenous sequence," as used herein, refers to a chromosomal sequence that is native to the cell.

As used herein, the term "exogenous" refers to a sequence that is not native to the cell, or a chromosomal sequence whose native location in the genome of the cell is in a different chromosomal location.

A "gene," as used herein, refers to a DNA region (including exons and introns) encoding a gene product, as well as all DNA regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites, and locus control regions.

The term "heterologous" refers to an entity that is not endogenous or native to the cell of interest. For example, a heterologous protein refers to a protein that is derived from or was originally derived from an exogenous source, such as an exogenously introduced nucleic acid sequence. In some instances, the heterologous protein is not normally produced by the cell of interest.

The term "nickase" refers to an enzyme that cleaves one strand of a double-stranded nucleic acid sequence (*i.e*., nicks a double-stranded sequence). For example, a nuclease with double strand cleavage activity can be modified by mutation and/or deletion to function as a nickase and cleave only one strand of a double-stranded sequence.

The term "nuclease," as used herein, refers to an enzyme that cleaves both strands of a double-stranded nucleic acid sequence.

The terms "nucleic acid" and "polynucleotide" refer to a deoxyribonucleotide or ribonucleotide polymer, in linear or circular conformation, and in either single- or double-stranded form. For the purposes of the present disclosure, these terms are not to be construed as limiting with respect to the length of a polymer. The terms can encompass known analogs of natural nucleotides, as well as nucleotides that are modified in the base, sugar and/or phosphate moieties (*e.g*., phosphorothioate backbones). In general, an analog of a particular nucleotide has the same base-pairing specificity; *i.e.*, an analog of A will base-pair with T.

The term "nucleotide" refers to deoxyribonucleotides or ribonucleotides. The nucleotides may be standard nucleotides (*i.e*., adenosine, guanosine, cytidine, thymidine, and uridine), nucleotide isomers, or nucleotide analogs. A nucleotide analog refers to a nucleotide having a modified purine or pyrimidine base or a modified ribose moiety. A nucleotide analog may be a naturally occurring nucleotide (*e.g*., inosine, pseudouridine, etc.) or a non-naturally occurring nucleotide. Non-limiting examples of modifications on the sugar or base moieties of a nucleotide include the addition (or removal) of acetyl groups, amino groups, carboxyl groups, carboxymethyl groups, hydroxyl groups, methyl groups, phosphoryl groups, and thiol groups, as well as the substitution of the carbon and nitrogen atoms of the bases with other atoms (*e.g*., 7-deaza purines). Nucleotide analogs also include dideoxy nucleotides, 2'-O-methyl nucleotides, locked nucleic acids (LNA), peptide nucleic acids (PNA), and morpholinos.

The terms "polypeptide" and "protein" are used interchangeably to refer to a polymer of amino acid residues.

The terms "target sequence," "target chromosomal sequence," and "target site" are used interchangeably to refer to the specific sequence in chromosomal DNA to which the engineered Cas9 system is targeted, and the site at which the engineered Cas9 system modifies the DNA or protein(s) associated with the DNA.

Techniques for determining nucleic acid and amino acid sequence identity are known in the art. Typically, such techniques include determining the nucleotide sequence of the mRNA for a gene and/or determining the amino acid sequence encoded thereby, and comparing these sequences to a second nucleotide or amino acid sequence. Genomic sequences can also be determined and compared in this fashion. In general, identity refers to an exact nucleotide-to-nucleotide or amino acid-to-amino acid correspondence of two polynucleotides or polypeptide sequences, respectively. Two or more sequences (polynucleotide or amino acid) can be compared by determining their percent identity. The percent identity of two sequences, whether nucleic acid or amino acid sequences, is the number of exact matches between two aligned sequences divided by the length of the shorter sequences and multiplied by 100. An approximate alignment for nucleic acid sequences is provided by the local homology algorithm of Smith and Waterman, Advances in Applied Mathematics 2:482-489 (1981). This algorithm can be applied to amino acid sequences by using the scoring matrix developed by Dayhoff, Atlas of Protein Sequences and Structure, M. O. Dayhoff ed., 5 suppl. 3:353-358, National Biomedical Research Foundation, Washington, D.C., USA, and normalized by Gribskov, Nucl. Acids Res. 14(6):6745-6763 (1986). An exemplary implementation of this algorithm to determine percent identity of a sequence is provided by the Genetics Computer Group (Madison, Wis.) in the "BestFit" utility application. Other suitable programs for calculating the percent identity or similarity between sequences are generally known in the art, for example, another alignment program is BLAST, used with default parameters. For example, BLASTN and BLASTP can be used using the following default parameters: genetic code=standard; filter=none; strand=both; cutoff=60; expect=10; Matrix=BLOSUM62; Descriptions=50 sequences; sort by=HIGH SCORE; Databases=non-redundant, GENBANK+EMBL+DDBJ+PDB+GENBANK CDS translations+Swiss protein+Spupdate+PIR. Details of these programs can be found on the GENBANK NIH genetic sequence database website.

Having described the invention in detail, it will be apparent that modifications and variations are possible without departing the scope of the invention defined in the appended claims. Furthermore, it should be appreciated that all examples in the present disclosure are provided as non-limiting examples.

### EXAMPLES

The following non-limiting examples are provided to further illustrate the present invention. It should be appreciated by those of skill in the art that the techniques disclosed in the examples that follow represent approaches the inventors have found function well in the practice of the invention, and thus can be considered to constitute examples of modes for its practice. Specific mutants described in the Examples below are part of the invention insofar as they include the mutations the selected from the group defined in claim 1.

### EXAMPLE 1: DIFFERENT AMINO ACID SUBSTITUTIONS ON K855 HAVE DIFFERENT ON-TARGET ACTIVITIES

The K855 residue of the wild-type SpCas9 was mutated to alanine, glutamic acid, isoleucine, methionine, or glutamine, and the recombinant proteins were purified from E. coli to over 95% homogeneity. The amino acid sequence for the K855Q mutant protein is listed on Table 1. All K855 mutant proteins share the same polypeptide sequence except for the K855 single mutation. A wild-type SpCas9 protein was purchased from MilliporeSigma for use as a control. A chemically synthesized HEKSite4 single guide RNA (sgRNA) with the guide sequence of 5'-GGCACUGCGGCUGGAGGUGG-3' (SEQ ID NO: 42) was also purchased from MilliporeSigma. Each protein was tested in three biological replicates.

Ribonucleoprotein (RNP) complexes were prepared by adding a buffer (20 mM HEPES, 100 mM KCI, 0.5 mM DTT, 0.1 mM EDTA, pH 7.5), 150 pmol sgRNA, and 8 µg of Cas9 protein to a 1.5-mL microcentrifuge tube in a 10 µL total reaction volume. The sgRNA to Cas9 protein molar ratio is approximately 3:1. The complexes were incubated at room temperature for 15 minutes and then kept on ice until transfection. Human U-2OS cells at approximately 80% confluency were detached with a trypsin solution and washed twice with Hank's Balanced Salt Solution. Cells were then resuspended in Nucleofector Solution V (Lonza) at approximately 0.25 × 10⁶ cells per 100 µL. Nucleofection was performed by transferring 100 µL of cells into RNP complexes and mixing immediately by gently pipetting up and down without introducing air bubbles before transferring into a cuvette for electroporation with Amaxa program X-001. Cells were immediately transferred to a 6-well plate containing 2 mL pre-warmed medium per well and grown at 37°C and 5% CO₂ for 3 days before being harvested for genomic modification assays.

Genomic DNA extracts from transfected cells were prepared using QuickExtract Solution. Targeted genomic regions were PCR amplified with next generation sequencing (NGS) primers using KAPA HiFi HotStart ReadyMix PCR Kit (Roche) with the following cycling condition: 95°C/3m; 98°C/20s, 68°C/30s, and 72°C/45s for 34 cycles; 72°C/5m. The NGS primers for the HEKSite4 target site were: 5'-TCGTCGGCAGCGTCAGATGTGTATAAGAGACAGNNNNNNGGAACCCAG GTAGCCAGAGA-3' (forward) (SEQ ID NO: 43) and 5'-GTCTCGTGGGCTCGGAGATGTGTATAAGAGACAGNNNNNNGGGGTGGG GTCAGACGT-3' (reverse) (SEQ ID NO: 44). The NGS primers for the HEKSite4 off-target site were: 5'-TCGTCGGCAGCGTCAGATGTGTATAAGAGACAGNNNNNNCTAGAGCAAA CCTTGGCATTGTCC-3' (forward) (SEQ ID NO: 45) and 5'-GTCTCGTGGGCTCGGAGATGTGTATAAGAGACAGNNNNNNACCCTCTAC CCTCCCTGATG-3' (reverse) (SEQ ID NO: 46). Primary PCR products were then reamplified with Illumina index primers using JumpStart^{™} Taq ReadyMix^{™} for Quantitative PCR Kit (MilliporeSigma) with the following cycling condition: 95°C/3m; 95°C/30s, 55°C/30s, and 72°C/30s for 8 cycles; 72°C/5m. Indexed PCR products were purified with Select-a-Size DNA Clean & Concentrator kit (Zymo) and quantified by PicoGreen (ThermoFisher). PCR products were then normalized and pooled to make NGS libraries. NGS was performed using an Illumina MiSeq instrument and a 2 × 300 bp kit. The FASTQ files for each sample were analyzed for genome editing frequency using an NGS analysis pipeline.

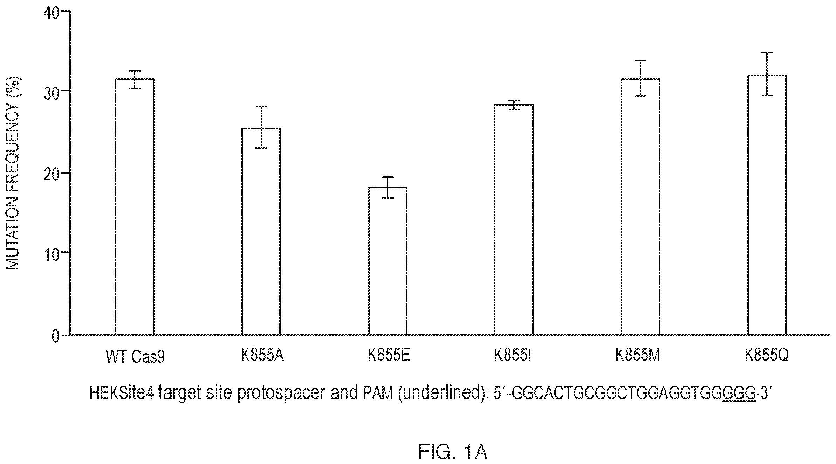

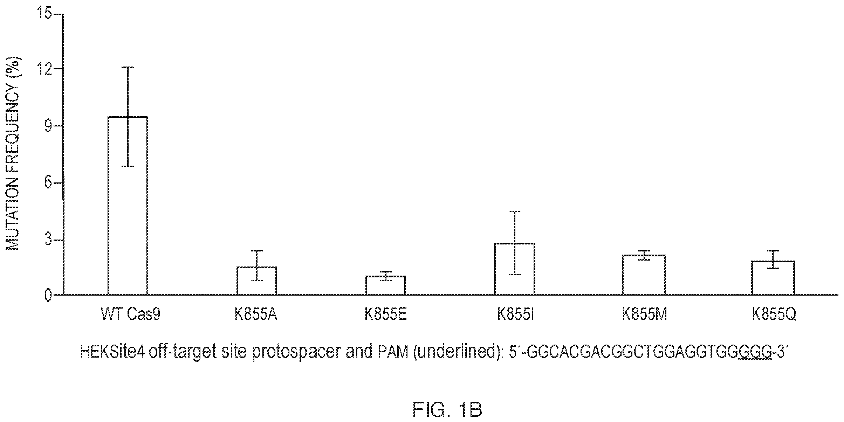

Results are presented in Figures 1A and 1B. The results show that different K855 mutant proteins had different levels of on-target activity and all five K855 mutant proteins substantially reduced off-target effects to a similar level. The results also show that glutamic acid and alanine were not an optimal substitution for K855 for maintaining the on-target activity.

**Table 1. SpCas9 K855Q amino acid sequence**

| |
|---|
| PAAKRVKLDGGGGSTGMDKKYSIGLDIGTNSVGWAVITDEYKVPSKKFKVLG TDRHSIKKNLIGALLFDSGETAEATRLKRTARRRYTRRKNRICYLQEIFSNEMAK VDDSFFHRLEESFLVEEDKKHERHPIFGNIVDEVAYHEKYPTIYHLRKKLVDSTD KADLRLIYLALAHMIKFRGHFLIEGDLNPDNSDVDKLFIQLVQTYNQLFEENPINA SGVDAKAILSARLSKSRRLENLIAQLPGEKKNGLFGNLIALSLGLTPNFKSNFDL AEDAKLQLSKDTYDDDLDNLLAQIGDQYADLFLAAKNLSDAILLSDILRVNTEITK APLSASMIKRYDEHHQDLTLLKALVRQQLPEKYKEIFFDQSKNGYAGYIDGGAS QEEFYKFIKPILEKMDGTEELLVKLNREDLLRKQRTFDNGSIPHQIHLGELHAILR RQEDFYPFLKDNREKIEKILTFRIPYYVGPLARGNSRFAWMTRKSEETITPWNF EEVVDKGASAQSFIERMTNFDKNLPNEKVLPKHSLLYEYFTVYNELTKVKYVTE GMRKPAFLSGEQKKAIVDLLFKTNRKVTVKQLKEDYFKKIECFDSVEISGVEDR FNASLGTYHDLLKIIKDKDFLDNEENEDILEDIVLTLTLFEDREMIEERLKTYAHLF DDKVMKQLKRRRYTGWGRLSRKLINGIRDKQSGKTILDFLKSDGFANRNFMQL IHDDSLTFKEDIQKAQVSGQGDSLHEHIANLAGSPAIKKGILQTVKVVDELVKVM GRHKPENIVIEMARENQTTQKGQKNSRERMKRIEEGIKELGSQILKEHPVENTQ LQNEKLYLYYLQNGRDMYVDQELDINRLSDYDVDHIVPQSFLKDDSIDNQVLTR SDKNRGKSDNVPSEEVVKKMKNYWRQLLNAKLITQRKFDNLTKAERGGLSEL DKAGFIKRQLVETRQITKHVAQILDSRMNTKYDENDKLIREVKVITLKSKLVSDF RKDFQFYKVREINNYHHAHDAYLNAVVGTALIKKYPKLESEFVYGDYKVYDVRK MIAKSEQEIGKATAKYFFYSNIMNFFKTEITLANGEIRKRPLIETNGETGEIVWDK GRDFATVRKVLSMPQVNIVKKTEVQTGGFSKESILPKRNSDKLIARKKDWDPKK YGGFDSPTVAYSVLVVAKVEKGKSKKLKSVKELLGITIMERSSFEKNPIDFLEAK GYKEVKKDLIIKLPKYSLFELENGRKRMLASAGELQKGNELALPSKYVNFLYLAS HYEKLKGSPEDNEQKQLFVEQHKHYLDEIIEQISEFSKRVILADANLDKVLSAYN KHRDKPIREQAENIIHLFTLTNLGAPAAFKYFDTTIDRKRYTSTKEVLDATLIHQSI TGLYETRIDLSQLGGDEFPKKKRKVGGGGSPKKKRKV (SEQ ID NO: 47) |
| Underline: Nuclear localization signal (NLS) |
| Bold Underline: K855Q mutation |

### EXAMPLE 2: DOUBLE MUTATION VARIANTS WITH OPTIMAL AMINO ACID SUBSTITUTIONS MAINTAIN ON-TARGET ACTIVITY

Different amino acid substitutions at R661, N692, or Q695 were introduced into the K855M and K855Q mutant backgrounds to generate double mutants. Recombinant proteins were purified from E. coli to over 95% homogeneity. All double mutants share the same polypeptide sequence as that of the K855Q mutant listed in Table 1 except for the designated mutations. Each protein was tested on the same HEKSite4 target site in U2-OS cells in three biological replicates. RNP complex preparation, cell transfection, and NGS analysis were as described in Example 1.

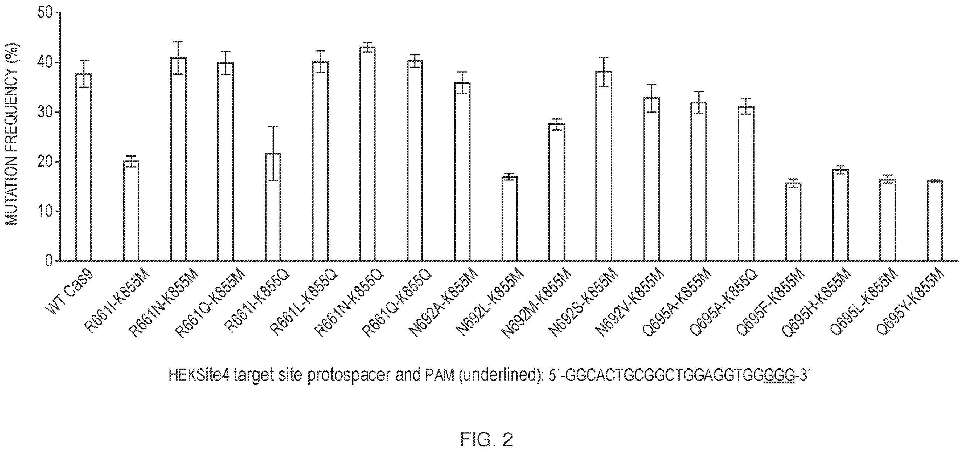

Results are presented in Figure 2. The results show that different amino acid substitutions at R661, N692, or Q695 resulted in different levels of on-target activity. On the R661 residue, isoleucine substitution resulted in a substantial reduction of the activity, whereas leucine, asparagine, or glutamine substitution maintained the same level of activity as that of WT Cas9. The substitution effects on the two non-charged residues were less predictable.

### EXAMPLE 3: TRIPLE MUTATION VARIANTS THAT ARE FEATURED WITH BALANCED SPECIFICITY AND ACTIVITY

A leucine or glutamine substitution at K526, K562, K652, R691, R780, K810, K848, K1003, or R1060 was introduced into the R661L-K855Q background to generate 18 triple mutants and one quadruple mutant (R661L-K855Q-K1003Q-R1060Q). All the triple and quadruple mutants share the same polypeptide sequence as that of the K855Q mutant listed in Table 1 except for the designated mutations. Recombinant proteins were purified from E. coli to over 95% homogeneity. Synthetic sgRNAs targeting human FANCF02 and HBB03 were purchased from MilliporeSigma. The guide sequences of these sgRNAs are listed in Table 2. eSpCas9 1.1 protein was purchased from MilliporeSigma and HiFi Cas9 V3 protein was purchased from Integrated DNA Technologies. Each protein was tested in three biological replicates.

RNP complexes were prepared as described in Example 1. Human k562 cells were seeded at 0.25 × 10⁶ cells per mL one day prior to transfection and were at approximately 0.5 × 10⁶ cells per mL at the time of transfection. Cells were washed twice with Hank's Balanced Salt Solution and then resuspended in Nucleofector Solution V (Lonza) at approximately 0.35 × 10⁶ cells per 100 µL. Nucleofection was performed by transferring 100 µL of cells into RNP complexes and mixing immediately by gently pipetting up and down without introducing air bubbles before transferring into a cuvette for electroporation with Amaxa program T-016. Cells were immediately transferred to a 6-well plate containing 2 mL pre-warmed medium per well and grown at 37°C and 5% CO₂ for 3 days before being harvested for genomic modification assays. Genomic DNA extracts from transfected cells were prepared using QuickExtract Solution. Targeted genomic regions were PCR amplified with NGS primers using JumpStart^{™} Taq ReadyMix^{™} for Quantitative PCR Kit (MilliporeSigma) with the following cycling condition: 98°C/2m; 98°C/15s, 62°C/30s, and 72°C/45s for 34 cycles; 72°C/5m. The NGS primer sequences are listed in Table 2. NGS library preparation, sequencing and data analysis were as described in Example 1.

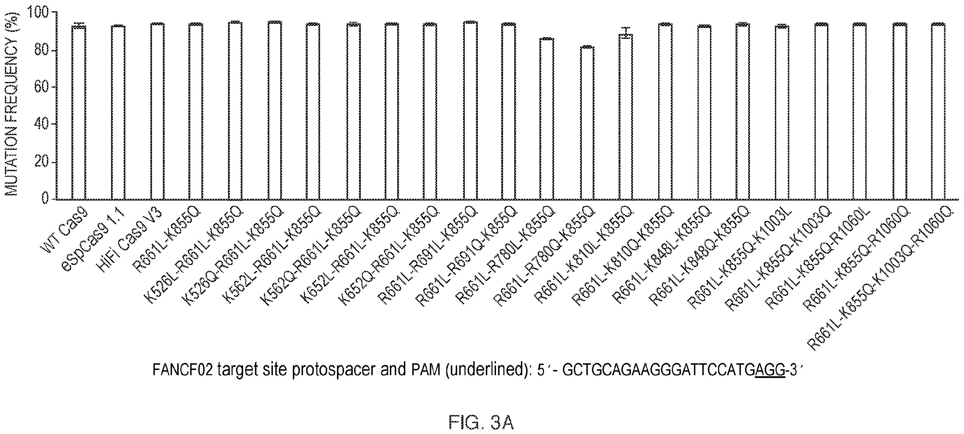

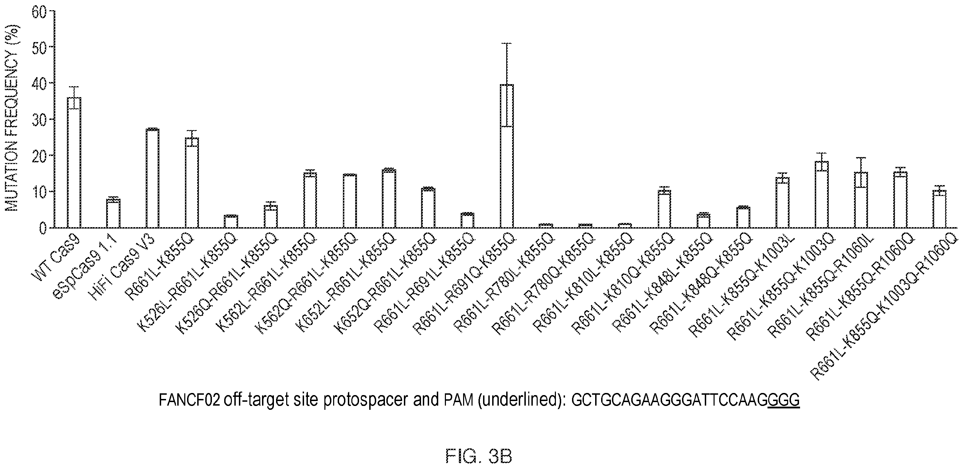

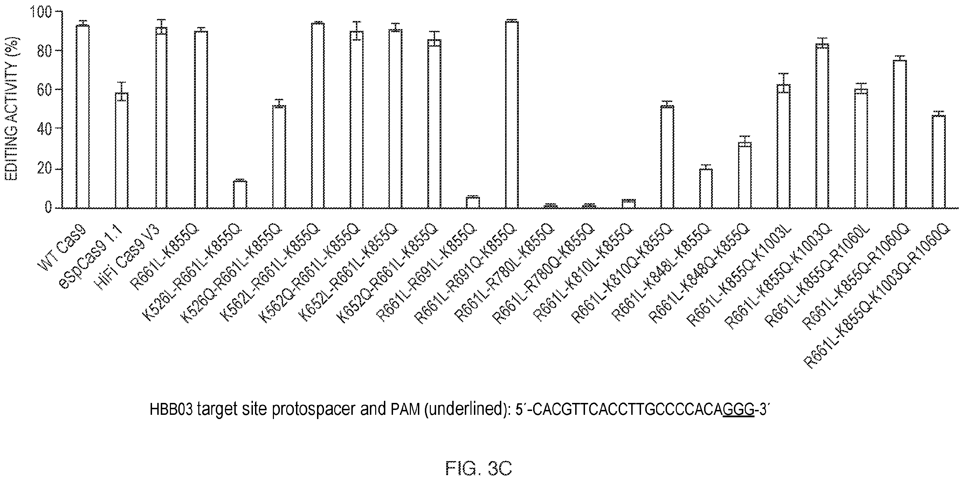

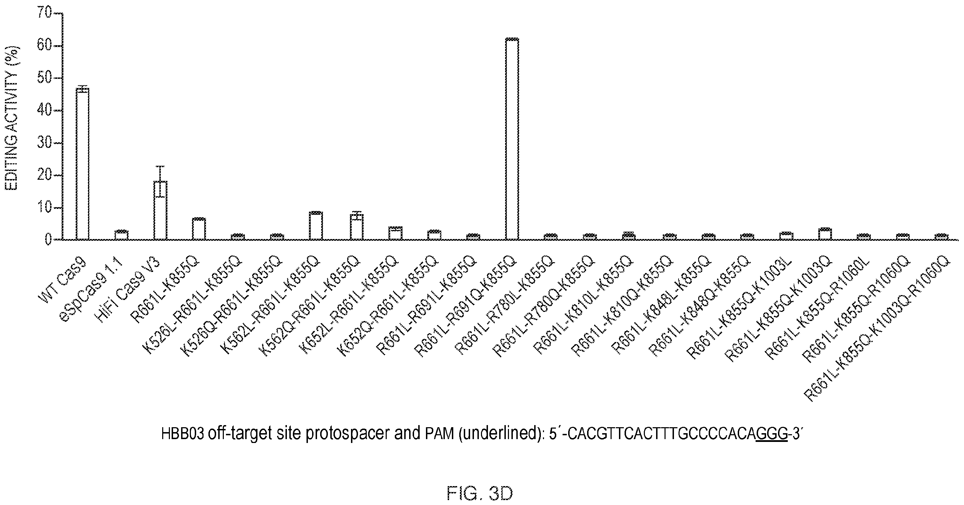

Results are presented in Figures 3A, 3B, 3C, and 3D. The results in Figures 3A and 3B show that all proteins were highly active on the FANCF02 target site and there was only a small variation among them. However, there was a wide range of variation among the proteins in the off-target mutation frequency at the FANCF02 single mismatch off-target site. Six triple mutant proteins were better than eSpCas9 1.1 in reducing the off-target effects. These include K526L-R661L-K855Q, R661L-R691L-K855Q, R661L-R780L-K855Q, R661L-R780Q-K855Q, R661L-K810L-K855Q, and R661L-K848L-K855Q. The remaining mutant proteins were either comparable to eSpCas9 1.1 or between eSpCas9 1.1 and HiFi Cas9 V3 in reducing the off-target mutation frequency, as compared with WT Cas9, except the outlier mutant R661L-R691Q-K855Q. The results in Figure 3C and 3D further differentiate the on-target activity and specificity levels among these proteins. The extremely high specific mutant proteins as identified on the FANCF02 site lost almost all on-target activities on the HBB03 site. However, six triple mutant proteins had a similar level of off-target mutation frequency to eSpCas9 1.1, but they had a substantially higher level of on-target activity than eSpCas9 1.1 on the HBB03 site. Based on the combined results, this group of mutant proteins were identified as having balanced specificity and activity. This selective group of mutant proteins include K562L-R661L-K855Q, K562Q-R661L-K855Q, K652L-R661L-K855Q, K652Q-R661L-K855Q, R661L-K855Q-K1003Q, and R661L-K855Q-R1060Q. Four eSpCas9 1.1-like triple mutant proteins were also identified based on the combined results, which include K526Q-R661L-K855Q, R661L-K810Q-K855Q, R661L-K855Q-K1003L, and R661L-K855Q-R1060L.

**Table 2. sgRNA guide sequences and NGS primers**

| Category | Sequence (5'-3') |
|---|---|
| FANCF0 2 sgRNA guide | GCUGCAGAAGGGAUUCCAUG (SEQ ID NO: 48) |
| HBB03 sgRNA guide | CACGUUCACCUUGCCCCACA (SEQ ID NO: 49) |
| FANCF0 2 target forward primer | TCGTCGGCAGCGTCAGATGTGTATAAGAGACAGNNNNNNAATGGGGCCATG CCGACCAA (SEQ ID NO: 50) |
| FANCF0 2 target reverse primer | GTCTCGTGGGCTCGGAGATGTGTATAAGAGACAGNNNNNNAGTTGCCCAGA GTCAAGGAACAC (SEQ ID NO: 51) |
| FANCF0 2 off-target forward primer | TCGTCGGCAGCGTCAGATGTGTATAAGAGACAGNNNNNNTCTTTCCCTCACT CTGGCTCG (SEQ ID NO: 52) |
| FANCF0 2 off-target reverse primer | GTCTCGTGGGCTCGGAGATGTGTATAAGAGACAGNNNNNNTGGAATGAATG GGGTGGGAGG (SEQ ID NO: 53) |
| HBB03 target forward primer | TCGTCGGCAGCGTCAGATGTGTATAAGAGACAGNNNNNNTAGGCAGAGAGA GTCAGTGCCTAT (SEQ ID NO: 54) |
| HBB03 target reverse primer | GTCTCGTGGGCTCGGAGATGTGTATAAGAGACAGNNNNNNCCAATCTACTCC CAGGAGCAG (SEQ ID NO: 55) |
| HBB03 off-target forward primer | TCGTCGGCAGCGTCAGATGTGTATAAGAGACAGNNNNNNTCACTGGAGCAG GGAGGACA (SEQ ID NO: 56) |
| HBB03 off-target reverse primer | GTCTCGTGGGCTCGGAGATGTGTATAAGAGACAGNNNNNNGGGTAGGAAAA CAGCCCAAGG (SEQ ID NO: 57) |

### EXAMPLE 4: SPECIFICITY IMPROVED SPCAS9 NUCLEASES MEDIATE EFFICIENT EDITING ACROSS DIFFERENT GENOMIC SITES

sgRNAs targeting five human genomic sites were purchased from MilliporeSigma. The guide sequences of these sgRNAs are listed in Table 3. RNP complexes were prepared as described in Example 1. Human K562 cells were seeded at 0.25 × 10⁶ cells per mL one day prior to transfection and were at approximately 0.5 × 10⁶ cells per mL at the time of transfection. Cells were washed twice with Hank's Balanced Salt Solution and then resuspended in Nucleofector Solution V (Lonza) at approximately 0.35 × 10⁶ cells per 100 µL. Nucleofection was performed by transferring 100 µL of cells into RNP complexes and mixing immediately by gently pipetting up and down without introducing air bubbles before transferring into a cuvette for electroporation with Amaxa program T-016. Cells were immediately transferred to a 6-well plate containing 2 mL pre-warmed medium per well and grown at 37°C and 5% CO₂ for 3 days before being harvested for genomic modification assays.

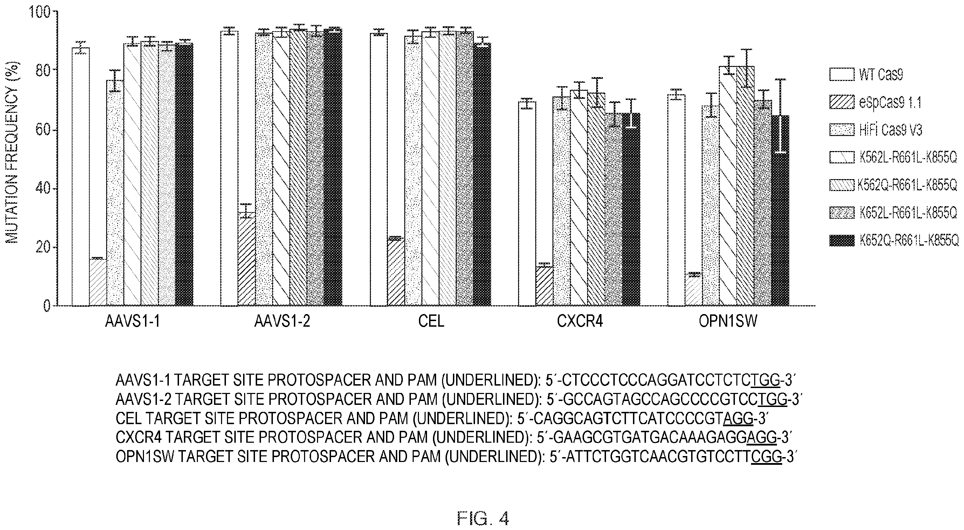

Genomic DNA extracts from transfected cells were prepared using QuickExtract Solution. Targeted genomic regions were PCR amplified with NGS primers using JumpStart^{™} Taq ReadyMix^{™} for Quantitative PCR Kit (MilliporeSigma) with the following cycling condition: 98°C/2m; 98°C/15s, 62°C/30s, and 72°C/45s for 34 cycles; 72°C/5m. The NGS primer sequences are listed in Table 3. NGS library preparation, sequencing and data analysis were as described in Example 1. Results are presented in Figure 4. The results show that the four triple mutant proteins identified with having balanced specificity and activity had substantially higher editing efficiencies than eSpCas9 1.1 and were comparable to WT Cas9 across all five genomic target sites.

**Table 3. sgRNA guide sequences and NGS primers**

| Category | Sequence (5'-3') |
|---|---|
| AAVS1-1 sgRNA guide | CUCCCUCCCAGGAUCCUCUC (SEQ ID NO: 58) |
| AAVS1-2 sgRNA guide | GCCAGUAGCCAGCCCCGUCC (SEQ ID NO: 59) |
| CEL sgRNA guide | CAGGCAGUCUUCAUCCCCGU (SEQ ID NO: 60) |
| CXCR4 guide | GAAGCGUGAUGACAAAGAGG (SEQ ID NO: 61) |
| OPN1SW guide | AUUCUGGUCAACGUGUCCUU (SEQ ID NO: 62) |
| AAVS1-1 NGS forward primer | TCGTCGGCAGCGTCAGATGTGTATAAGAGACAGNNNNNNCTTGGGAAGTGT AAGGAAGCTGC (SEQ ID NO: 63) |
| AAVS1-1 NGS reverse primer | GTCTCGTGGGCTCGGAGATGTGTATAAGAGACAGNNNNNNGCCTCCTCCTT CCTAGTCTCC (SEQ ID NO: 64) |
| AAVS1-2 NGS forward primer | TCGTCGGCAGCGTCAGATGTGTATAAGAGACAGNNNNNNGCTGCAGCTTCC TTACACTTCC (SEQ ID NO: 65) |
| AAVS1-2 NGS reverse primer | GTCTCGTGGGCTCGGAGATGTGTATAAGAGACAGNNNNNNGAGGAATATGT CCCAGATAGCACT (SEQ ID NO: 66) |
| CEL NGS forward primer | TCGTCGGCAGCGTCAGATGTGTATAAGAGACAGNNNNNNCTGTGGGATTTC ATGGAAGTTCAGC (SEQ ID NO: 67) |
| CEL NGS reverse primer | GTCTCGTGGGCTCGGAGATGTGTATAAGAGACAGNNNNNNATCCTGGCTG GCAAGGTGG (SEQ ID NO: 68) |
| CXCR4 NGS forward primer | TCGTCGGCAGCGTCAGATGTGTATAAGAGACAGNNNNNNTTCCTTGGCCTC TGACTGTTG (SEQ ID NO: 69) |
| CXCR4 NGS reverse primer | GTCTCGTGGGCTCGGAGATGTGTATAAGAGACAGNNNNNNTTCCTGCCCAC CATCTACTCC (SEQ ID NO: 70) |
| OPN1SW NGS forward | TCGTCGGCAGCGTCAGATGTGTATAAGAGACAGNNNNNNGATGGGCCTCA GTACCACATTG (SEQ ID NO: 71) |
| primer OPN1SW NGS reverse primer | GTCTCGTGGGCTCGGAGATGTGTATAAGAGACAGNNNNNNCAACCTTTGCC TTCCCCTAACC (SEQ ID NO: 72) |

## Claims

1. An engineered *Streptococcus pyogenes* Cas9 (SpCas9) protein variant having an identity of at least 99% identity with SEQ ID NO:1 and comprising the mutations selected from the following group:
K562L-R661L-K855Q;
K562Q-R661L-K855Q;
K652L-R661L-K855Q;
K652Q-R661L-K855Q;
R661L-K855Q-K1003Q;
R661L-K855Q-R1060Q;
R661L-R691L-K855Q;
R661L-R780L-K855Q;
R661L-R780Q-K855Q;
R661L-K81 0L-K855Q;
R661L-K848L-K855Q;
R661L-K81 0Q-K855Q;
R661L-K855Q-K1003L; and
R661L-K855Q-R1060L;
with reference to the amino acid numbering in the corresponding position of an unmodified mature, wild-type *Streptococcus pyogenes* Cas9 shown in SEQ ID NO:1.

2. The engineered SpCas9 protein variant of claim 1, wherein the mutations are K652Q-R661L-K855Q.

3. The engineered SpCas9 protein variant of any preceding claim, further comprising one or more heterologous domains fused to the N terminal end, the C terminal end, an internal location, or combination thereof and/or at least one nuclear localization signal fused to the N terminal end, the C terminal end, an internal location, or combination thereof.

4. The engineered SpCas9 protein variant of claim 3, wherein the heterologous domain is selected from a nuclear localization signal, a cell-penetrating domain, a marker or reporter domain which facilitates detection, a chromatin modification domain, an epigenetic modification domain, a transcriptional regulation domain, a DNA or RNA deaminase domain, a uracil-DNA-glycosylase domain, a reverse transcriptase domain, a recombinase domain, an RNA aptamer binding domain, and a non-Cas9 nuclease domain.

5. The engineered SpCas9 protein variant of any preceding claim, further comprising at least one mutation in the RuvC domain, and/or at least one mutation in the HNH domain, optionally wherein the at least one mutation in the RuvC domain, if present, comprises at least one mutation selected from D10A, D8A, E762A, and D986A, and optionally wherein the at least one mutation in the HNH domain, if present, comprises at least one mutation selected from H840A, H559A, N854A, N856A, and N863A.

6. An engineered Cas9 system comprising the engineered SpCas9 protein variant of any preceding claim and at least one engineered guide RNA, wherein the at least one engineered guide RNA is designed to complex with the engineered SpCas9 protein variant.

7. A plurality of nucleic acids encoding the engineered SpCas9 protein variant of any of claims 1-5 or the engineered SpCas9 system of claim 6.

8. The plurality of nucleic acids of claim 7, wherein the plurality of nucleic acids comprises at least one nucleic acid encoding the engineered SpCas9 protein variant, optionally codon optimized for expression in a eukaryotic cell, and at least one nucleic acid encoding the engineered guide RNA.

9. The plurality of nucleic acids of claim 7 or 8, wherein:
(a) at least one nucleic acid is RNA;
(b) at least one nucleic acid is DNA;
(c) the at least one nucleic acid encoding the engineered guide RNA is DNA;
(d) the at least one nucleic acid encoding the engineered SpCas9 protein variant is operably linked to a phage promoter sequence for *in vitro* RNA synthesis or protein expression in a bacterial cell, and the at least one nucleic acid encoding the engineered guide RNA is operably linked to a phage promoter sequence for *in vitro* RNA synthesis; or
(e) the at least one nucleic acid encoding the engineered Cas9 protein variant is operably linked to a eukaryotic promoter sequence for expression in a eukaryotic cell, and the at least one nucleic acid encoding the engineered guide RNA is operably linked to a eukaryotic promoter sequence for expression in a eukaryotic cell.

10. The plurality of nucleic acids of claim 8 or 9, wherein the eukaryotic cell is a human cell, a non-human mammalian cell, a non-mammalian vertebrate cell, an invertebrate cell, a plant cell, or a single cell eukaryotic organism.

11. At least one vector comprising the plurality of nucleic acids of any one of claims 7-10, which is optionally a plasmid vector, a viral vector, or a self-replicating viral RNA replicon.

12. A eukaryotic cell comprising at least one engineered Cas9 system of claim 6 or the nucleic acid of any of claims 7-10, wherein the cell is optionally a human cell, a non-human mammalian cell, a plant cell, a non-mammalian vertebrate cell, an invertebrate cell, or a single cell eukaryotic organism.

13. The eukaryotic cell of claim 12, which is *ex vivo,* or *in vitro.*

14. The engineered SpCas9 protein variant of any of claims 1-5, which is a Cas9 homologue.

15. A ribonucleoprotein (RNP) complex comprising the engineered SpCas9 protein variant of any of claims 1-5.

16. A fusion protein comprising the engineered SpCas9 protein variant of any of claims 1-5.

17. A pharmaceutical composition comprising the engineered SpCas9 protein variant of any of claims 1-5 and at least one pharmaceutically acceptable excipient.

18. A method for modifying the chromosomal sequence of a eukaryotic cell, the method comprising expressing in the eukaryotic cell the engineered SpCas9 protein variant of any of claims 1-5 together with a guide RNA,
wherein the method is not a process for modifying the germ line genetic identity of human beings and
wherein the method is not a method for treatment of the human or animal body by surgery or therapy.

## Patentansprüche

1. Konstruierte Proteinvariante von *Streptococcus pyogenes* Cas9 (SpCas9), die eine Identität von zumindest 99 % Identität mit SEQ ID NO: 1 aufweist und die Mutationen umfasst, die aus der folgenden Gruppe ausgewählt sind:
K562L-R661L-K855Q;
K562Q-R661L-K855Q;
K652L-R661L-K855Q;
K652Q-R661L-K855Q;
R661L-K855Q-K1003Q;
R661L-K855Q-R1060Q;
R661L-R691L-K855Q;
R661L-R780L-K855Q;
R661L-R780Q-K855Q;
R661L-K810L-K855Q;
R661L-K848L-K855Q;
R661L-K810Q-K855Q;
R661L-K855Q-K1003L; und
R661L-K855Q-R1060L;
unter Bezugnahme auf die Aminosäurenummerierung an der entsprechenden Position eines unmodifizierten reifen Wildtyps von *Streptococcus pyogenes* Cas9, der in SEQ ID NO: 1 gezeigt ist.

2. Konstruierte SpCas9-Proteinvariante nach Anspruch 1, wobei die Mutationen K652Q-R661L-K855Q sind.

3. Konstruierte SpCas9-Proteinvariante nach einem vorhergehenden Anspruch, ferner umfassend eine oder mehrere heterologe Domänen, die an das N-terminale Ende, das C-terminale Ende, eine interne Stelle oder Kombination davon kondensiert sind, und/oder zumindest ein Kernlokalisierungssignal, das an das N-terminale Ende, das C-terminale Ende, eine interne Stelle oder Kombination davon kondensiert ist.

4. Konstruierte SpCas9-Proteinvariante nach Anspruch 3, wobei die heterologe Domäne ausgewählt ist aus einem Kernlokalisierungssignal, einer zellpenetrierenden Domäne, einer Marker- oder Reporterdomäne, die Erfassung erleichtert, einer Chromatinmodifikationsdomäne, einer epigenetischen Modifikationsdomäne, einer Transkriptionsregulationsdomäne, einer DNA- oder RNA-Desaminasedomäne, einer Uracil-DNA-Glycosylasedomäne, einer Reverse-Transkriptase-Domäne, einer Rekombinasedomäne, einer RNA-Aptamer-Bindungsdomäne und einer Nicht-Cas9-Nukleasedomäne.

5. Konstruierte SpCas9-Proteinvariante nach einem vorhergehenden Anspruch, ferner umfassend zumindest eine Mutation in der RuvC-Domäne und/oder zumindest eine Mutation in der HNH-Domäne, wobei optional die zumindest eine Mutation in der RuvC-Domäne, falls vorhanden, zumindest eine Mutation ausgewählt aus D10A, D8A, E762A und D986A umfasst und wobei optional die zumindest eine Mutation in der HNH-Domäne, falls vorhanden, zumindest eine Mutation ausgewählt aus H840A, H559A, N854A, N856A und N863A umfasst.

6. Konstruiertes Cas9-System, das die konstruierte SpCas9-Proteinvariante nach einem vorhergehenden Anspruch und zumindest eine konstruierte Führungs-RNA umfasst, wobei die zumindest eine konstruierte Führungs-RNA gestaltet ist, um einen Komplex mit der konstruierten SpCas9-Proteinvariante zu bilden.

7. Vielzahl von Nukleinsäuren, welche die konstruierte SpCas9-Proteinvariante nach einem der Ansprüche 1-5 oder das konstruierte SpCas9-System nach Anspruch 6 kodiert.

8. Vielzahl von Nukleinsäuren nach Anspruch 7, wobei die Vielzahl von Nukleinsäuren zumindest eine Nukleinsäure, welche die konstruierte SpCas9-Proteinvariante kodiert, optional Codon-optimiert für Expression in einer eukaryotischen Zelle, und zumindest eine Nukleinsäure, welche die konstruierte Führungs-RNA kodiert, umfasst.

9. Vielzahl von Nukleinsäuren nach Anspruch 7 oder 8, wobei:
(a) zumindest eine Nukleinsäure RNA ist;
(b) zumindest eine Nukleinsäure DNA ist;
(c) die zumindest eine Nukleinsäure, welche die konstruierte Führungs-RNA kodiert, DNA ist;
(d) die zumindest eine Nukleinsäure, welche die konstruierte SpCas9-Proteinvariante kodiert, mit einer Phagen-Promotorsequenz für *In-vitro*-RNA-Synthese oder Proteinexpression in einer Bakterienzelle wirkverknüpft ist, und die zumindest eine Nukleinsäure, welche die konstruierte Führungs-RNA kodiert, mit einer Phagen-Promotorsequenz für *In-vitro*-RNA-Synthese wirkverknüpft ist; oder
(e) die zumindest eine Nukleinsäure, welche die konstruierte Cas9-Proteinvariante kodiert, mit einer eukaryotischen Promotorsequenz für Expression in einer eukaryotischen Zelle wirkverknüpft ist, und die zumindest eine Nukleinsäure, welche die konstruierte Führungs-RNA kodiert, mit einer eukaryotischen Promotorsequenz für Expression in einer eukaryotischen Zelle wirkverknüpft ist.

10. Vielzahl von Nukleinsäuren nach Anspruch 8 oder 9, wobei die eukaryotische Zelle eine menschliche Zelle, eine nicht-menschliche Säugerzelle, eine Nicht-Säuger-Wirbeltierzelle, eine Wirbellosen-Zelle, eine Pflanzenzelle oder ein einzelliger eukaryotischer Organismus ist.

11. Zumindest ein Vektor, der die Vielzahl von Nukleinsäuren nach einem der Ansprüche 7-10 umfasst, der optional ein Plasmidvektor, ein viraler Vektor oder ein selbstreplizierendes virales RNA-Replikon ist.

12. Eukaryotische Zelle, die zumindest ein konstruiertes Cas9-System nach Anspruch 6 oder die Nukleinsäure nach einem der Ansprüche 7-10 umfasst, wobei die Zelle optional eine menschliche Zelle, eine nicht-menschliche Säugerzelle, eine Pflanzenzelle, eine Nicht-Säuger-Wirbeltierzelle, eine Wirbellosen-Zelle oder ein einzelliger eukaryotischer Organismus ist.

13. Eukaryotische Zelle nach Anspruch 12, die *ex vivo* oder *in vitro* ist.

14. Konstruierte SpCas9-Proteinvariante nach einem der Ansprüche 1-5, die ein Cas9-Homolog ist.

15. Ribonukleoprotein(RNP-)Komplex, der die konstruierte SpCas9-Proteinvariante nach einem der Ansprüche 1-5 umfasst.

16. Fusionsprotein, das die konstruierte SpCas9-Proteinvariante nach einem der Ansprüche 1-5 umfasst.

17. Pharmazeutische Zusammensetzung, welche die konstruierte SpCas9-Proteinvariante nach einem der Ansprüche 1-5 und zumindest einen pharmazeutisch verträglichen Hilfsstoff umfasst.

18. Verfahren zum Modifizieren der Chromosomensequenz einer eukaryotischen Zelle, wobei das Verfahren Exprimieren der konstruierten SpCas9-Proteinvariante nach einem der Ansprüche 1-5 zusammen mit einer Führungs-RNA in der eukaryotischen Zelle umfasst,
wobei das Verfahren kein Prozess zum Modifizieren der genetischen Identität der Keimbahn von Menschen ist, und
wobei das Verfahren kein Verfahren zur Behandlung des menschlichen oder tierischen Körpers durch Operation oder Therapie ist.

## Revendications

1. Variant de protéine Cas9 de *Streptococcus pyogenes* (SpCas9) modifié comportant une identité d'au moins 99 % avec la SEQ ID n° : 1 et comprenant les mutations choisies dans le groupe suivant :
K562L-R661L-K855Q ;
K562Q-R661L-K855Q ;
K652L-R661L-K855Q ;
K652Q-R661L-K855Q ;
R661L-K855Q-K1003Q ;
R661L-K855Q-R1060Q ;
R661L-R691L-K855Q ;
R661L-R780L-K855Q ;
R661L-R780Q-K855Q ;
R661L-K810L-K855Q ;
R661L-K848L-K855Q ;
R661L-K810Q-K855Q ;
R661L-K855Q-K1003L ; et
R661L-K855Q-R1060L ;
en référence à la numérotation des acides aminés dans la position correspondante d'une Cas9 de *Streptococcus pyogenes* de type sauvage mature non modifiée présentée dans la SEQ ID n° : 1.

2. Variant de protéine SpCas9 modifié selon la revendication 1, lesdites mutations étant K652Q-R661L-K855Q.

3. Variant de protéine SpCas9 modifié selon l'une quelconque des revendications précédentes, comprenant en outre un ou plusieurs domaines hétérologues fusionnés à l'extrémité N terminale, l'extrémité C terminale, un emplacement interne ou une combinaison de ceux-ci et/ou au moins un signal de localisation nucléaire fusionné à l'extrémité N terminale, l'extrémité C terminale, un emplacement interne ou une combinaison de ceux-ci.

4. Variant de protéine SpCas9 modifié selon la revendication 3, ledit domaine hétérologue étant choisi parmi un signal de localisation nucléaire, un domaine de pénétration cellulaire, un domaine marqueur ou rapporteur qui facilite la détection, un domaine de modification de chromatine, un domaine de modification épigénétique, un domaine de régulation de transcription, un domaine d'ADN ou ARN désaminase, un domaine uracile-ADN-glycosylase, un domaine transcriptase inverse, un domaine recombinase, un domaine de liaison d'aptamère d'ARN et un domaine sans Cas9 nucléase.

5. Variant de protéine SpCas9 modifié selon l'une quelconque des revendications précédentes, comprenant en outre au moins une mutation dans le domaine RuvC, et/ou au moins une mutation dans le domaine HNH, éventuellement ladite au moins une mutation dans le domaine RuvC, s'il est présent, comprenant au moins une mutation choisie parmi D10A, D8A, E762A et D986A, et éventuellement ladite au moins une mutation dans le domaine HNH, s'il est présent, comprenant au moins une mutation choisie parmi H840A, H559A, N854A, N856A et N863A.

6. Système de Cas9 modifié comprenant le variant de protéine SpCas9 modifié selon l'une quelconque des revendications précédentes et au moins un ARN guide modifié, ledit au moins un ARN guide modifié étant conçu pour se complexer avec le variant de protéine SpCas9 modifié.

7. Pluralité d'acides nucléiques codant le variant de protéine SpCas9 modifié selon l'une quelconque des revendications 1 à 5 ou système SpCas9 modifié selon la revendication 6.

8. Pluralité d'acides nucléiques selon la revendication 7, ladite pluralité d'acides nucléiques comprenant au moins un acide nucléique codant le variant de protéine SpCas9 modifié, éventuellement un codon optimisé pour l'expression dans une cellule eucaryote, et au moins un acide nucléique codant l'ARN guide modifié.

9. Pluralité d'acides nucléiques selon la revendication 7 ou 8 :
(a) au moins un acide nucléique étant de l'ARN ;
(b) au moins un acide nucléique étant de l'ADN ;
(c) ledit au moins un acide nucléique codant l'ARN guide modifié étant de l'ADN ;
(d) ledit au moins un acide nucléique codant le variant de protéine SpCas9 modifié étant lié fonctionnellement à une séquence de promoteur de phage pour la synthèse d'ARN *in vitro* ou l'expression de protéines dans une cellule bactérienne, et ledit au moins un acide nucléique codant l'ARN guide modifié étant lié fonctionnellement à une séquence de promoteur de phage pour la synthèse d'ARN *in vitro* ; ou
(e) ledit au moins un acide nucléique codant le variant de protéine SpCas9 modifié étant lié fonctionnellement à une séquence de promoteur eucaryote pour l'expression dans une cellule eucaryote, et ledit au moins un acide nucléique codant l'ARN guide modifié étant lié fonctionnellement à une séquence de promoteur eucaryote pour l'expression dans une cellule eucaryote.

10. Pluralité d'acides nucléiques selon la revendication 8 ou 9, ladite cellule eucaryote étant une cellule humaine, une cellule de mammifère non humaine, une cellule de vertébré non mammifère, une cellule d'invertébré, une cellule végétale ou un organisme eucaryote unicellulaire.

11. Au moins un vecteur comprenant la pluralité d'acides aminés selon l'une quelconque des revendications 7 à 10, qui est éventuellement un vecteur plasmidique, un vecteur viral ou un réplicon d'ARN viral auto-répliquant.

12. Cellule eucaryote comprenant au moins un système de Cas9 modifié selon la revendication 6 ou l'acide nucléique selon l'une quelconque des revendications 7 à 10, ladite cellule étant éventuellement une cellule humaine, une cellule de mammifère non humaine, une cellule végétale, une cellule de vertébré non mammifère, une cellule d'invertébré ou un organisme eucaryote unicellulaire.

13. Cellule eucaryote selon la revendication 12, qui est *ex vivo* ou *in vitro.*

14. Variant de protéine SpCas9 modifié selon l'une quelconque des revendications 1 à 5, qui est un homologue de Cas9.

15. Complexe de ribonucléoprotéine (RNP) comprenant le variant de protéine SpCas9 modifié selon l'une quelconque des revendications 1 à 5.

16. Protéine de fusion comprenant le variant de protéine SpCas9 modifié selon l'une quelconque des revendications 1 à 5.

17. Composition pharmaceutique comprenant le variant de protéine SpCas9 modifié selon l'une quelconque des revendications 1 à 5 et au moins un excipient pharmaceutiquement acceptable.

18. Procédé permettant la modification de la séquence chromosomique d'une cellule eucaryote, le procédé comprenant l'expression dans la cellule eucaryote du variant de protéine SpCas9 modifié selon l'une quelconque des revendications 1 à 5 conjointement à un ARN guide,
ledit procédé n'étant pas un procédé destiné à modifier l'identité génétique de lignée germinale d'êtres humains et
ledit procédé n'étant pas un procédé destiné au traitement du corps humain ou animal par chirurgie ou thérapie.
